# FASCICULE DE BREVET EUROPEEN

(11) **EP 1 248 859 B1**
(45) Date de publication et mention de la délivrance du brevet: **30.03.2005**
(21) Numéro de dépôt: 01907607.4
(22) Date de dépôt: 17.01.2001
(51) Int. Cl.: C12Q 1/68, B01J 19/00

(54) **PROCEDES DE SYNTHESE ET D'IMMOBILISATION D'ACIDES NUCLEIQUES SUR UN SUPPORT SOLIDE SILANISE**
VERFAHREN ZUR HERSTELLUNG UND IMMOBILISIERUNG VON NUKLEINSÄURE AUF EINEM SILAN-MODIFIZIERTEN FESTTRÄGER
METHOD FOR SYNTHESISING AND IMMOBILISING NUCLEIC ACIDS ON A SILANIZED SOLID SUPPORT

(30) Priorité: 20.01.2000 FR 0000697
(43) Date de publication de la demande: 16.10.2002
(73) Titulaire: CENTRE NATIONAL DE LA RECHERCHE SCIENTIFIQUE (CNRS), 75016 Paris (FR); ECOLE CENTRALE DE LYON, 69131 Ecully Cédex (FR)
(72) Inventeur: BENNETAU, Bernard, F-33400 Talence (FR); BOUSBAA, Jamal, F-33405 Talence Cedex (FR); CHOPLIN, Franck, F-72320 Valennes (FR); SOUTEYRAND, Eliane, F-69130 Ecully (FR); MARTIN, Jean-René, F-69380 Lozanne (FR); CLOAREC, Jean-Pierre, F-69001 Lyon (FR)
(74) Mandataire: Goulard, Sophie
(86) Numéro de dépôt international: PCT/FR2001/000140
(87) Numéro de publication internationale: WO 2001/053523

(56) Documents cités:
- L. A. CHRISEY, G. U. LEE, E. O'FERRALL: "Covalent attachment of synthetic DNA to self-assembled monolayer films" NUCLEIC ACIDS RESEARCH, vol. 24, no. 15, 1996, pages 3031-3039, XP002149193 cité dans la demande
- N. BALACHANDER, C. N. SUKENIK: "Functionalized siloxy-anchored monolayers with exposed amino, azido, bromo, or cyano groups" TETRAHEDRON LETTERS, vol. 29, no. 44, 1988, pages 5593-5594, XP002149194
- CHRISEY L A ET AL: "FABRICATION OF PATTERNED DNA SURFACES" NUCLEIC ACIDS RESEARCH,GB,OXFORD UNIVERSITY PRESS, SURREY, vol. 24, no. 15, 1996, pages 3040-3047, XP002913337 ISSN: 0305-1048
- F. EFFENBERGER, S. HEID: "Synthesis of model compounds for the formation of self-assembled monolayers on a silicon surface" SYNTHESIS, 1995, pages 1126-1130, XP002149195

## Description

La présente invention se rapporte à l'utilisation, pour la synthèse ou l'immobilisation d'acides nucléiques, d'un support solide modifié par une monocouche auto assemblée organisée d'un ou de plusieurs composés organosiliciés, ainsi qu'à des procédés de synthèse et d'immobilisation d'acides nucléiques sur un support solide.

Pour effectuer des synthèses chimiques ou immobiliser des molécules sur une surface minérale, il est tout d'abord nécessaire de greffer sur celle-ci des agents de couplage qui vont assurer la fixation des molécules organiques sur le substrat minéral.

Des agents de couplage organosiliciés ont été proposés dans ce but par L.A. CHRISEY *et al*. (*Nucleic Acids Research*, 1996, *24,* 15, 3031-3039) et U. MASKOS *et al*. (*Nucleic Acids Research,* 1992, *20,* 7, 1679-1684). Les agents utilisés, à savoir le 3-glycidoxypropyltriméthoxysilane et différents aminosilanes, présentent toutefois l'inconvénient de se déposer de façon aléatoire et non reproductible sur la surface. Ils y forment un film inhomogène dont l'épaisseur ne peut être contrôlée, film qui est en outre peu robuste vis-à-vis de traitements chimiques ultérieurs, l'inhomogénéité du film impliquant en effet une mauvaise protection des liaisons siloxaniques. Il est donc très difficile d'obtenir un greffage reproductible de ces molécules. Avant de fixer ou de synthétiser des oligonucléotides sur le substrat, des réactions de surface supplémentaires sont nécessaires pour diminuer la gène stérique à la surface (par exemple greffage de molécules hétérocycliques bifonctionnelles, comme décrit par L.A. CHRISEY *et al.*), rendre la surface plus hydrophile (U. MASKOS *et al*. décrivent le greffage d'éthylène glycol, de penta- ou d'hexa-éthylèneglycol) et/ou pallier la faible réactivité des fonctions de surface, opérations supplémentaires qui, elles aussi, ne sont pas maîtrisées.

A. ULMAN a décrit, dans *Chem*. *Rev.,* 1996, *96*, 1533-1554, la formation de monocouches autoassemblées organisées sur des supports solides à l'aide de composés organosiliciés de type alkyltrichlorosilanes fonctionnalisés. Leur utilisation pour la fixation de biomolécules est proposée, méthode qui nécessite vraissemblablement, dans ce contexte, une modification de la biomolécule par une fonction thiol et une modification de la surface par des molécules hétérobifonctionnelles.

Ainsi, les Inventeurs se sont donné pour but de pallier les inconvénients de l'art antérieur et de pourvoir à des procédés de synthèse et d'immobilisation de biomolécules, notamment d'acides nucléiques, sur des supports solides modifiés par des films monocouches denses et organisés.

La présente invention a pour objet l'utilisation, pour la synthèse ou l'immobilisation d'acides nucléiques, d'un support solide modifié par une monocouche autoassemblée organisée comprenant au moins un composé organosilicié répondant à la formule (I) : 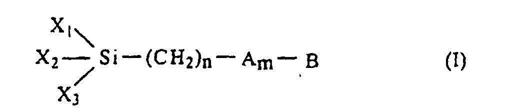 dans laquelle:
- n est compris entre 15 et 35, de préférence entre 20 et 25
- m est égal à 0 ou à 1,
- X₁, X₂ et X₃, qui peuvent être identiques ou différents entre eux, sont sélectionnés dans le groupe constitué par les groupes alkyles saturés en C₁ à C₆, linéaires ou ramifiés, et les groupements hydrolysables, au moins l'un de X₁, X₂ ou X₃ représentant un groupement hydrolysable,
- A représente le groupement -O-(CH₂-CH₂-O)ₖ-(CH₂)ᵢ- dans lequel k est compris entre 0 et 100, de préférence entre 0 et 5, et i représente un nombre entier supérieur ou égal à 0, de préférence égal à 0 ou à 1,
- dans le cas où m = 0, alors B représente un groupement -OCOR, -OR, -COOR ou un atome d'halogène, R représentant un atome d'hydrogène ou un groupe alkyle en C₁ à C₆, linéaire ou ramifié,
- dans le cas où m = 1 :
   . si k = 0 et i = 0, alors B représente un groupement R₁,
   . si k = 0 et i ≥ 1, alors B représente un groupement -OR₁, -OCOR₁, -NR₁R₂, -COOR₁, -CONR₁R₂, -SR₁ ou un atome d'halogène,
   . si k ≥ 1 et i = 0, alors B représente un groupement -R₁, -COR₁, -COOR₁ ou -CONR₁R₂,
   . si k ≥ 1 et i ≥ 1, alors B représente un groupement -OR₁, -OCOR₁, -NR₁R₂, -COOR₁, -CONR₁R₂, -SR₁ ou un atome d'halogène,
   . R₁ et R₂, qui peuvent être identiques ou différents, représentent un atome d'hydrogène, une chaîne hydrocarbonée éventuellement substituée, saturée ou insaturée et linéaire ou ramifiée, comprenant de 1 à 24 atomes de carbone, ou un groupement aromatique.

Lorsque B représente un groupement -OR₁, -OCOR₁ ou -COOR₁, indifféremment des valeurs de i, et lorsque k ≥ 1, alors il est bien entendu que B peut représenter tout groupement résultant de la protection d'une fonction hydroxyle ou acide carboxylique, tels que les groupements protecteurs décrits dans *Protective groups in organic synthesis* (T.W. GREENE *et al.,* 2^{nd} edition, Wiley Interscience), par exemple un groupement protecteur cyclique.

Au sens de la présente invention et dans ce qui suit, on entend par « acides nucléiques » aussi bien des oligonucléotides que des ADN ou des ARN, ou encore des acides nucléiques au squelette ou aux bases modifiés, tels que les acides nucléiques peptidiques (PNA : Peptide Nucleic Acids) qui font intervenir des liaisons peptidiques à la place des liaisons phosphodiesters.

On entend par « aromatique » tout groupement qui possède un ou plusieurs noyaux aryles, par exemple un noyau phényle. On entend par « monocouche autoassemblée organisée » un assemblage de molécules dans lequel les molécules sont organisées, organisation due à des interactions et à une forte cohésion entre les chaînes des molécules, donnant lieu à un film anisotrope stable et ordonné (A. ULMAN, *Chem*. *Rev*., 1996, *96*, 1533-1554).

Une monocouche autoassemblée organisée formée sur un support solide permet l'obtention d'une surface organique dense, homogène et de paramètres bien définis à la fois chimiquement et structurellement. La formation de cette monocouche, obtenue grâce aux propriétés d'autoassemblage des composés de formule (I) pour des valeurs de n, m, k et i bien définies, est parfaitement reproductible pour chaque composé organosilicié. En outre, la formation d'une monocouche autoassemblée organisée, très dense, protège les liaisons siloxaniques vis-à-vis des traitements chimiques (acides ou basiques), ce qui permet de réaliser des réactions chimiques variées sur cette surface.

Les composés organosiliciés de formule (I) utilisés dans la présente invention présentent avantageusement des fonctionnalités très variées et une grande réactivité, eu égard à la nature du groupe A et à la diversité des groupements B terminaux utilisables, ces groupements B pouvant bien entendu être modifiés et fonctionnalisés à volonté selon les réactions de chimie organique bien connues de l'Homme du métier.

L'utilisation selon la présente invention permet, de façon particulièrement avantageuse et de par les composés organosiliciés de formule (I) sélectionnés, de synthétiser ou d'immobiliser des acides nucléiques sur un support de façon fiable et reproductible, eu égard à l'homogénéité et à la stabilité de la monocouche autoassemblée organisée formée sur le support. Les acides nucléiques sont synthétisés ou immobilisés sur le support modifié par des liaisons covalentes fortes, sans dégradation des liaisons siloxaniques développées entre les composés organosiliciés et le support solide.

Des supports solides convenables sont, de manière générale, ceux dont la surface est hydratée et/ou ceux dont la surface présente des groupements hydroxyles. De préférence, ledit support est sélectionné dans le groupe constitué par les verres, les céramiques (par exemple de type oxyde), les métaux (par exemple l'aluminium ou l'or) et les métalloïdes (tel que le silicium).

Au sens de la présente invention, on entend par « hydrolysable » tout groupe capable de réagir avec un acide en milieu aqueux de façon à donner les composés X₁H, X₂H ou X₃H, X₁, X₂ et X₃ étant tels que définis dans la formule (I).

Selon un mode de mise en oeuvre avantageux, ledit groupement hydrolysable est sélectionné dans le groupe constitué par les atomes d'halogène, le groupement -N(CH₃)₂ et les groupements -OR', R' étant un groupe alkyle saturé en C₁ à C₆, linéaire ou ramifié.

En ce qui concerne les groupements B et les groupements hydrolysables, des atomes d'halogène convenables sont aussi bien le fluor que le chlore, le brome ou l'iode.

Selon un autre mode de mise en oeuvre avantageux, X₁, X₂ et X₃ représentent des atomes de chlore, n est égal à 22, m est égal à 1, i est égal à 0, k est égal à 1 ou à 3 et B représente un groupement -COCH₃.

Selon un autre mode de mise en oeuvre avantageux, X₁, X₂ et X₃ représentent des atomes de chlore, n est égal à 22, m est égal à 1, i est égal à 1, k est égal à 2 et B représente un groupement -COOCH₃.

Selon un autre mode de mise en oeuvre avantageux, X₁, X₂ et X₃ représentent des atomes de chlore, n est égal à 16, 22 ou 27, m est égal à 0 et B représente un groupement -OCOCH₃.

Selon encore un autre mode de mise en oeuvre avantageux, X₁, X₂ et X₃ représentent des atomes de chlore, n est égal à 21, m est égal à 0 et B représente un groupement -COOCH₃.

L'utilisation de supports solides modifiés selon la présente invention est particulièrement avantageuse pour la préparation de puces à ADN, à savoir de supports sur lesquels sont fixés, de façon covalente, un ensemble d'ADN de séquences connues dans un ordre bien précis, ces puces étant réutilisables de nombreuses fois. De telles puces à ADN permettent, par hybridation des ADN immobilisés sur le support avec des acides nucléiques ou des oligonucléotides cibles, de déterminer la séquence de ces molécules cibles ou de suivre l'expression de gènes. Les applications sont nombreuses : découverte de nouveaux gènes, de nouveaux médicaments, réalisation de diagnostics, études de toxicité, etc.

La présente invention a également pour objet un procédé de synthèse d'acides nucléiques sur un support solide, caractérisé en ce que ledit support est modifié par une monocouche autoassemblée organisée comprenant au moins un composé organosilicié répondant à la formule (I) telle que définie précédemment, et en ce qu'il comprend les étapes suivantes :
a) préparation d'un support solide modifié par une monocouche autoassemblée organisée comprenant au moins un composé organosilicié répondant à la formule (I) telle que définie précédemment, dans lequel lesdits composés organosiliciés présentent à leur extrémité une fonction hydroxyle ou amine protégée ;
b) éventuellement, déprotection de la fonction hydroxyle ou amine ;
c) couplage covalent, de façon localisée, d'un nucléotide sur le support solide modifié obtenu à l'étape a) ou b) ;
d) réitération de l'étape c) au moins une fois, avec des nucléotides identiques ou différents.

L'étape a) est avantageusement réalisée par les étapes suivantes :
i) élimination des contaminants du support solide et hydratation et/ou hydroxylation de sa surface,
j) introduction dans un mélange d'au moins deux solvants comprenant au moins un solvant hydrocarboné non polaire, sous atmosphère inerte, d'un composé organosilicié de formule générale (I) telle que définie ci-dessus, ledit composé présentant à une extrémité une fonction hydroxyle ou amine protégée,
k) silanisation du support obtenu à l'étape i) par immersion dans la solution préparée à l'étape j),
l) éventuellement, recuit du support silanisé obtenu à l'étape k), portant la monocouche autoassemblée, à une température comprise entre 50 et 120°C, pour une durée de 5 minutes à une nuit, et
m) rinçage du support modifié obtenu à l'étape k) ou l) à l'aide d'un solvant, de préférence polaire.

Par « contaminants » du support solide, on entend tout composé tel que de la graisse, des poussières ou autres, présent à la surface du support et qui ne fait pas partie de la structure chimique du support lui-même.

Selon la nature du support solide, l'étape i) peut être effectuée à l'aide d'un ou de plusieurs solvants et/ou oxydants et/ou hydroxylants (par exemple un mélange sulfochromique), d'un détergents (par exemple du Hellmanex®), d'un traitement photochimique à l'ozone ou tout autre traitement approprié.

Lors de l'étape j), à titre d'exemples de fonctions hydroxyles ou amines protégées, on peut citer les groupements -OCOR' et -NR'R", dans lesquels R' et R" représentent des chaînes alkyles. Tout autre groupe résultant de la protection d'une fonction hydroxyle ou amine est également envisageable.

L'étape j) peut avantageusement être réalisée dans un mélange d'au moins un solvant hydrocarboné non polaire et d'au moins un solvant polaire. Dans ce cas, les proportions volumiques de solvant non polaire et de solvant polaire sont de préférence comprises entre 70/30 et 95/5.

A titre d'exemples et de façon non limitative, au cours de l'étape j), un solvant hydrocarboné non polaire utilisable est le cyclohexane et un solvant polaire utilisable est le chloroforme.

Lors de l'étape j), la concentration du composé organosilicié dans le mélange de solvants est de préférence comprise entre 1.10⁻⁵ et 1.10⁻² mole/litre.

L'étape k) de silanisation du support peut être effectuée pendant un temps compris entre 1 minute et 3 jours et à une température comprise entre -10°C et 120°C selon les solvants utilisés.

Au cours du procédé de synthèse d'acides nucléiques sur un support solide selon la présente invention, l'étape b) peut être réalisée par toute technique connue de l'Homme du métier, par exemple par un traitement à la potasse. Les étapes c) et d) peuvent être réalisées par la chimie des phosphoramidites ou par toutes autres chimies permettant le couplage covalent de nucléotides connues de l'Homme du métier, décrites par exemple par A. ELLINGTON et J.D. POLLARD dans *Current Protocols in Molecular Biology,* John Wiley & Sons Inc., New York.

Selon un mode de mise en oeuvre avantageux du procédé de synthèse selon l'invention, une étape de greffage, à l'extrémité desdits composés organosiliciés, de bras espaceurs portant des fonctions hydroxyles ou amines terminales est effectuée après l'étape a) ou b) et avant l'étape c).

De tels bras espaceurs peuvent être de charge, de longueur et de polarité variables selon les propriétés souhaitées. Par exemple, ils peuvent comprendre une chaîne chargée (tel qu'un phosphoramidite avec un site abasique), une chaîne hydrophile non chargée (tel qu'un polyéthylène glycol), une chaîne hydrophobe non chargée (telle qu'une chaîne alkyle), ou encore une chaîne comprenant un groupement labile à l'ammoniaque (tel que le groupement -SO₂).

L'étape c) de couplage covalent d'un nucléotide sur le support modifié est réalisée de façon localisée, c'est-à-dire en un endroit déterminé du support.

La réitération de l'étape c) peut être réalisée autant de fois que souhaité ; on peut par exemple effectuer l'étape c) une centaine de fois, sachant que le rendement diminue au-delà d'un tel nombre.

Les nucléotides couplés aux étapes c) et d) sont de préférence convenablement protégés, tel que décrit dans la littérature relative à des synthèses oligonucléotidiques sur supports solides. Ultérieurement à l'étape d), il est bien entendu que les acides nucléiques synthétisés sur le support solide peuvent être déprotégés par tout traitement chimique approprié, par exemple de l'ammoniaque. Ce traitement ne s'accompagne pas d'un clivage des acides nucléiques du support, sauf si un bras espaceur labile à l'ammoniaque a été introduit entre ces acides nucléiques et la surface silanisée.

La présente invention a également pour objet un procédé d'immobilisation d'acides nucléiques sur un support solide, caractérisé en ce que ledit support est modifié par une monocouche autoassemblée organisée comprenant au moins un composé organosilicié répondant à la formule (I) telle que définie précédemment, et en ce qu'il comprend les étapes suivantes :
a) préparation d'un support solide modifié par une monocouche autoassemblée organisée comprenant au moins un composé organosilicié répondant à la formule (I) telle que définie précédemment, dans lequel lesdits composés organosiliciés présentent à leur extrémité une fonction acide carboxylique ou amine protégée ;
b) éventuellement, déprotection de la fonction acide carboxylique ou amine ;
c) éventuellement, dans le cas où le support solide modifié porte des fonctions acides carboxyliques terminales, activation de ces fonctions ;
d) mise en contact du support solide modifié obtenu à l'étape a), b) ou c) avec une ou plusieurs solutions appliquées localement, dans un ou plusieurs solvants polaires, du(des) acide(s) nucléique(s) à immobiliser, lesdits acides nucléiques portant à l'une de leurs extrémités un bras espaceur fonctionnalisé soit par une fonction amine, dans le cas où le support solide modifié porte des fonctions acides carboxyliques terminales éventuellement protégées, soit par une fonction acide carboxylique activée, dans le cas où le support solide modifié porte des fonctions amines terminales éventuellement protégées ; et
e) lavage du support solide sur lequel sont immobilisés lesdits acides nucléiques.

L'étape a) est avantageusement réalisée comme indiqué précédemment en rapport avec le procédé de synthèse d'acides nucléiques sur un support solide, à la différence près que, lors de l'étape j), le composé organosilicié présente à une extrémité une fonction acide carboxylique ou amine protégée.

L'étape c) d'activation des fonctions acides carboxyliques peut par exemple être effectuée à l'aide d'une solution de N-hydroxysuccinimide ou de carbodiimide, ou encore tout autre réactif d'activation convenable et connu de l'Homme du métier.

Au cours de l'étape d), il est bien entendu que, pour solubiliser les acides nucléiques, toute solution permettant une bonne solubilité de ces derniers et un contrôle de l'évaporation de la solution pourrait être utilisée ; on peut citer, à titre d'exemple et de façon non limitative, un mélange acétonitrile/eau. Chaque solution d'acide nucléique à immobiliser est déposée en un endroit déterminé du support par un moyen adapté de microdéposition.

Selon un mode de mise en oeuvre avantageux du procédé d'immobilisation selon l'invention, une étape de greffage, à l'extrémité desdits composés organosiliciés, de bras espaceurs portant des fonctions acides carboxyliques ou amines terminales est effectuée après l'étape a) ou b) et avant l'étape c). Des bras espaceurs convenables sont tels que décrits ci-avant.

La présente invention a, en outre, pour objet une puce à ADN, caractérisée en ce qu'elle est obtenue par le procédé d'immobilisation d'acides nucléiques selon la présente invention, dans lequel lesdits acides nucléiques sont des ADN.

La puce à ADN selon l'invention présente l'avantage d'être stable et donc de pouvoir être réutilisée dans de nombreux cycles d'hybridation et de dénaturation.

Outre les dispositions qui précèdent, l'invention comprend encore d'autres dispositions qui ressortiront de la description qui va suivre, qui se réfère à des exemples de synthèse de composés organosiliciés utilisables dans le cadre de la présente invention et de synthèse ou d'immobilisation d'acides nucléiques sur des supports solides modifiés par une monocouche autoassemblée organisée de ces composés organosiliciés, ainsi qu'aux dessins annexés, dans lesquels :
- les figures 1, 2 et 3 illustrent la synthèse de précurseurs insaturés des composés organosiliciés de formule (I) dans laquelle m = 1,
- la figure 4 illustre la silylation de ces précurseurs insaturés,
- la figure 5 représente des précurseurs insaturés de composés organosiliciés de formule (I) dans laquelle m = 0,
- la figure 6 représente des spectres infra-rouge réalisés après trois expériences de greffage du composé organosilicié 14 sur la surface silice de substrats Au/Si/SiO₂,
- la figure 7a représente la densité, analysée par spectroscopie Raman, de la surface du substrat Au/Si/SiO₂ sur lequel sont greffés les composés organosiliciés 14 ; les figures 7b et 7c représentent les spectres Raman pris en deux points différents de cette surface, et
- la figure 8 représente des expériences successives d'hybridation (figures 8d, 8f et 8h) et de dénaturation (figures 8e, 8g et 8i) à partir de substrats Au/Si/SiO₂ modifiés par une monocouche de composés organosiliciés de formule (I) sur lequel les oligonucléotides L 185 et U226 ont été synthétisés (figure 8a).

Il doit être bien entendu, toutefois, que ces exemples sont donnés uniquement à titre d'illustration de l'objet de l'invention, dont ils ne constituent en aucune manière une limitation.

### EXEMPLE 1 : Synthèse de composés organosiliciés de formule (I) dans laquelle m = 1.

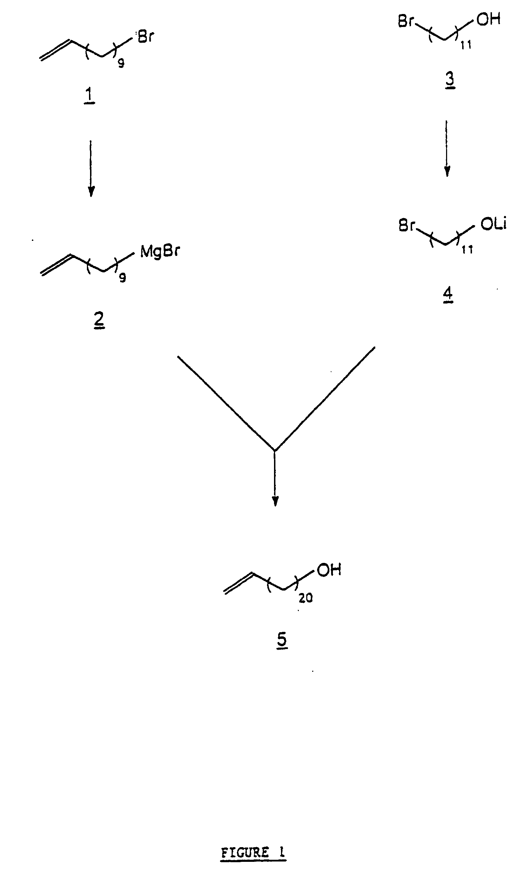

### 1) Synthèse d'un alcool insaturé (figure 1).

### • Préparation du magnésien 2

Dans un ballon tricol de 500 ml, sous atmosphère inerte, est introduit du magnésium (1,8 g; 70 mmol). Le dérivé bromé insaturé 1 (16,3 g; 70 mmol), préalablement mis en solution dans 70 ml de THF (tétrahydrofurane) anhydre, est ajouté goutte à goutte. Quelques gouttes de dibromoéthane peuvent être nécessaires pour activer le magnésium. Le mélange réactionnel est porté au reflux pendant 1 h 30 afin d'obtenir le magnésien 2, qui sera utilisé immédiatement.

### • Préparation de l'alcoolate de lithium 4

Dans un ballon tricol sec de 250 ml, sous atmosphère inerte, le bromo-alcool 3 (17,7 g; 70 mmol; 1 éq.) est mis en solution dans 70 ml de THF anhydre. La solution est refroidie à -78°C puis du méthyllithium (50 ml; 80 mmol; 1,1 éq.) est ajouté goutte à goutte. On obtient l'alcoolate de lithium 4.

### • Préparation de l'alcool insaturé 5

Le magnésien 2 est refroidi à -78°C, puis de l'iodure de cuivre (1,1 g; 3,5 mmol; 0,05 éq.) est ajouté. La solution est agitée pendant 25 minutes à -78°C, puis réchauffée à température ambiante jusqu'à obtenir une couleur pourpre. La solution est alors immédiatement refroidie à -78°C et l'alcoolate de lithium 4 est introduit à l'aide d'une canule sous atmosphère d'argon. La solution est agitée pendant 1 h à -78°C puis pendant 18 h à température ambiante. L'excès de méthyllithium est détruit par addition d'éthanol, suivie d'une hydrolyse en milieu acide par addition d'une solution aqueuse d'acide chlorydrique à 10%. La phase organique est extraite trois fois à l'éther diéthylique. Les phases éthérées sont rassemblées, lavées avec une solution d'acide chlorhydrique à 10%, à l'eau et enfin avec une solution aqueuse saturée en NaHCO₃. La phase organique est ensuite lavée jusqu'à neutralité, séché sur MgSO₄ puis concentrée sous vide. Le produit est purifié par reprécipitation dans l'acétone. Le composé 5 est obtenu sous la forme d'un solide blanc (19,8 g; point de fusion de 61,7-62,8°C; rendement de 87%). Son analyse par infra-rouge et RMN du proton et du carbone 13 est la suivante.
IR (ν (cm⁻¹)); 3330; 3079; 2919; 2851; 1642.
RMN ¹H (CDCl₃; δ (ppm)): 6,0-5,7 (m; 1 H); 5,1-4,7 (m; 2 H); 3,6 (t; 2 H); 2,2-1,9 (m; 2 H) et 1,7-1,2 (m; 37 H dont 1 H échangeable à D₂O).
RMN ¹³C (CDCl₃; δ (ppm)): 139,3; 113,8; 62,8; 33,6-25,8 (19 CH₂).

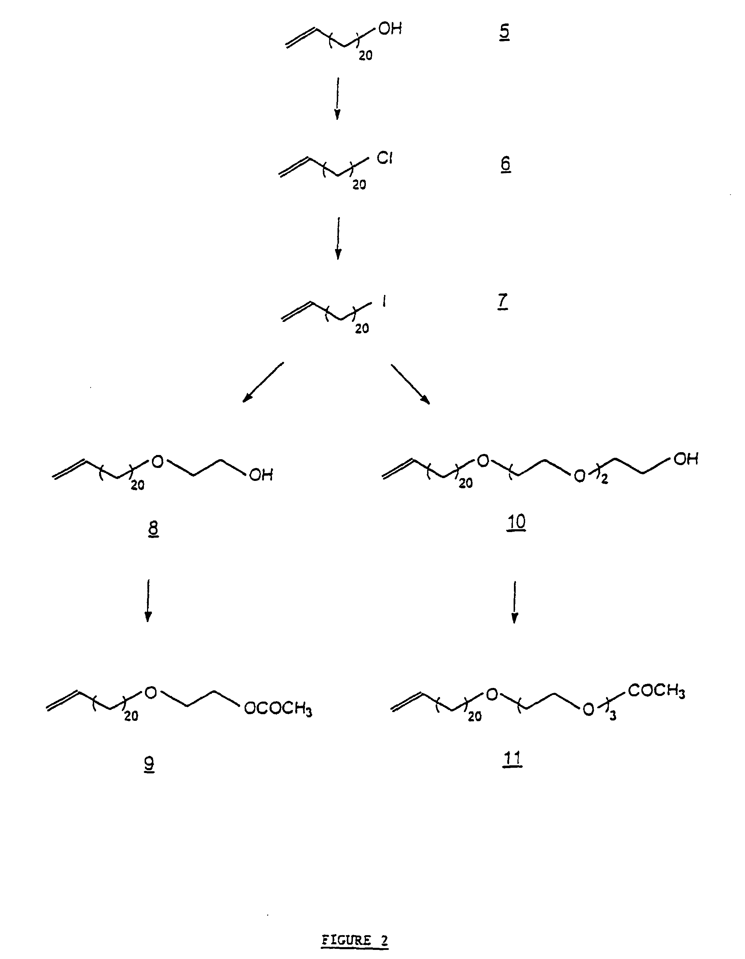

### 2) Introduction d'un éthylène glycol (figure 2).

### • Synthèse du dérivé chloré insaturé 6

L'alcool 5 obtenu précédemment (15 g; 46 mmol; 1 éq.) et de la pyridine (40,36 ml; 6 mmol; 0,1 éq.) sont introduits dans un ballon bicol de 250 ml, équipé d'une agitation magnétique et surmonté d'un réfrigérant ascendant. Du chlorure de thionyle (6 ml; 70 mmol; 1,5 éq.) est alors ajouté goutte à goutte. Le milieu réactionnel est agité pendant 1 h puis est porté au reflux jusqu'à la disparition complète de la bande OH (suivie par spectroscopie infra-rouge). Le milieu réactionnel est ensuite hydrolysé puis extrait trois fois à l'éther diéthylique. Les phases éthérées sont rassemblées, lavées avec une solution d'acide chlorhydrique à 10%, à l'eau, puis avec une solution saturée en NaHCO₃. La phase éthérée est ensuite lavée jusqu'à neutralité, séchée sur MgSO₄ et concentrée sous vide. Le composé 6 est obtenu sous la forme d'un solide jaune, puis est purifié par chromatographie sur silice (éluant: éther de pétrole/éther, v/v 70/30); un solide blanc est obtenu (14 g; point de fusion de 34,1-34,9°C; rendement de 75%). Son analyse par infra-rouge et RMN du proton et du carbone 13 est la suivante.
IR (dispersion dans KBr) ν (cm⁻¹): 3076; 2917; 2849; 1641.
RMN ¹H (CDCl₃; δ (ppm)): 6,0-5,7 (m; 1 H); 5,1-4,7 (m; 2 H); 3,5-3,3 (t; 2 H); 2,2-1,9 (m; 2 H) et 1,7-1,2 (m; 36 H).
RMN ¹³C (CDCl₃, δ (ppm)): 139,2; 113,8; 33,8-25,6 (20 CH₂).

### • Synthèse du dérivé iodé insaturé 7

Dans un ballon de 250 ml, le composé chloré insaturé 6 (10,6g; 32 mmol) et de l'iodure de sodium (22 g; 140 mmol; 4 éq.) sont solubilisés dans de l'acétone (40 ml). La solution est alors portée au reflux pendant 18 h. Le milieu réactionnel est ensuite extrait à l'éther diéthylique, les phases éthérées sont rassemblées puis lavées à l'eau, séchées sur MgSO₄ et concentrées sous vide. Le produit est purifié par des précipitations dans l'acétone. Le composé 7 est obtenu sous la forme d'un solide jaune (11g; point de fusion de 41,1-42,0°C; rendement de 81%). Son analyse par infra-rouge et RMN du proton et du carbone 13 est la suivante.
IR (dispersion dans KBr) ν (cm⁻¹): 3076; 2917; 2849; 1641.
RMN ¹H (CDCl₃; δ ppm)): 6,0-5,7 (m; 1 H); 5,1-4,7 (m; 2 H); 3,2-3,0 (t; 2 H); 2,2-1,9 (m; 2 H) et 1,7-1,2 (m; 36 H).
RMN ¹³C (CDCl₃; δ (ppm)): 139,2; 113,8; 33,8-25,6 (20 CH₂).

### • Synthèse de l'alcool insaturé 8

Une solution d'éthylène glycol (11,5 g; 180 mmol; 10 éq.) et de soude préalablement réduite en poudre (3,7 g; 90 mmol; 5 éq.) dans 20 ml de THF anhydre est portée au reflux durant 30 minutes. Le composé iodé 1 (8 g; 18 mmol; 1 éq) et du tétrabutyl-hydrogénosulfate d'ammonium (0,62 g; 1,8 mmol; 0,1 éq.) sont ajoutés. Le milieu réactionnel est ensuite porté au reflux durant 72 h. Après retour à température ambiante, une solution aqueuse d'acide chlorhydrique (10%, 50 ml) est introduite. Le milieu réactionnel est ensuite extrait trois fois à l'éther diéthylique; les phases éthérées sont rassemblées, lavées deux fois avec une solution d'acide chlorhydrique à 10%, à l'eau, puis avec une solution saturée en NaHCO₃. La phase éthérée est ensuite lavée jusqu'à neutralité, séchée sur MgSO₄ et concentrée sous vide. Le solide obtenu est reprécipité dans le dichlorométhane, puis purifié par chromatographie sur silice (éluant: dichlorométane/acétate d'éthyle; v/v: 30/70). Le composé 8 est obtenu sous la forme d'un solide blanc (1,4 g, point de fusion de 61,2-62,4°C; rendement de 21%). Son analyse par infra-rouge et RMN du proton et du carbone 13 est la suivante.
IR (dispersion dans KBr) ν (cm⁻¹): 3330; 3080; 2917; 2849; 1643.
RMN ¹H (CDCl₃; δ (ppm)): 6,0-5,7 (m; 1 H); 5,1-4,7 (m; 2 H); 3,75-3,65 (m; 2H) 3,55-3,4 (m; 4 H); 2,2-1,9 (m; 2 H) et 1,7-1,0 (m; 37 H dont 1 H échangeable à D₂O).
RMN ¹³C (CDCl₃; δ (ppm)): 139,2; 113,8; 71,7 (2 CH₂); 63,7; 33,6-25,8 (19 CH₂).

### • Synthèse de l'alcool insaturé protégé sous forme d'ester 9

Dans un ballon bicol de 100 ml, l'alcool insaturé 8 (0,9 g; 2,7 mmol) est mis en suspension dans du dichlorométhane (10 ml) et de la triéthylamine (0,6 ml; 5,4 mmol; 2 éq.). Le milieu réactionnel est refroidi à 0°C puis du chlorure d'acétyle (0,5 ml; 4 mmol; 1,5 éq.) est ajouté goutte à goutte à l'aide d'une seringue. Le milieu réactionnel est agité pendant 15 minutes à 0°C, puis 1 h 30 à température ambiante. Il est ensuite hydrolysé, puis extrait trois fois à l'éther diéthylique. Les phases éthérées sont rassemblées, lavées avec une solution d'acide chlorhydrique à 10%, à l'eau, puis avec une solution saturée en NaHCO₃. La phase éthérée est alors lavée jusqu'à neutralité, séchée sur MgSO₄ et concentrée sous vide. Le composé 9 est obtenu sous la forme d'un solide blanc (0,9 g; rendement de 100%). Son analyse par infra-rouge et RMN du proton et du carbone 13 est la suivante.
IR (dispersion dans KBr) ν (cm⁻¹): 3080; 2917; 2849; 1742; 1643.
RMN ¹H (CDCl₃; δ (ppm)): 6,0-5,7 (m; 1 H); 5,1-4,7 (m; 2 H); 4,25-4,15 (m; 2H); 3,60-3,50 (t; 2 H); 3,45-3,35 (t; 2 H); 2,2-1,9 (m; 5 H) et 1,7-1,0 (m; 36 H).
RMN ¹³C (CDCl₃; δ (ppm)): 172,0; 139,2; 113,8; 71,5; 68,5; 63,7; 33,6-25,8 (19 CH₂); 21,0.

### 3) Introduction de trois motifs éthylène glycol (figure 2).

A partir du dérivé iodé insaturé 7 obtenu ci-dessus et de triéthylèneglycol, un alcool insaturé 10 est obtenu, puis estérifié pour obtenir le produit 11, et ce selon les mêmes protocoles qu'exposés ci-dessus.

L'analyse du produit 10 par infra-rouge et RMN du proton et du carbone 13 est la suivante. IR (dispersion dans KBr) ν (cm⁻¹): 3380; 3079; 2919; 2850; 1641. RMN ¹H (CDCl₃; δ (ppm)): 5,9-5,7 (m; 1 H); 5,1-4,7 (m; 2 H); 4,3-4,2 (t; 2H); 3,7-3,4 (m; 10 H); 3,4-3,3 (t; 2 H); 2,2-1,9 (m; 2 H) et 1,7-1,0 (m; 37 H dont 1 H échangeable à D₂O). RMN ¹³C (CDCl₃; δ (ppm)): 139,3; 114,0; 71,6-68,2 (6 CH₂); 63,6; 33,6-25,8 (19 CH₂).

L'analyse du produit 11 par infra-rouge et RMN du proton et du carbone 13 est la suivante. IR (dispersion dans KBr) ν (cm⁻¹): 3079; 2919; 2850; 1740; 1641, RMN ¹H (CDCl₃; δ (ppm)): 5,9-5,7 (m; 1 H); 5,1-4,7 (m; 2 H); 4,3-4,2 (t; 2H); 3,7-3,4 (m; 10 H); 3,4-3,3 (t; 2 H); 2,2-1,9 (m; 5 H) et 1,7-1,0 (m; 36 H). RMN ¹³C (CDCl₃; δ (ppm)): 171,5; 139,3; 114,0; 71,6-68,3 (6 CH₂); 63,6; 33,6-25,8 (19 CH₂); 21,0.

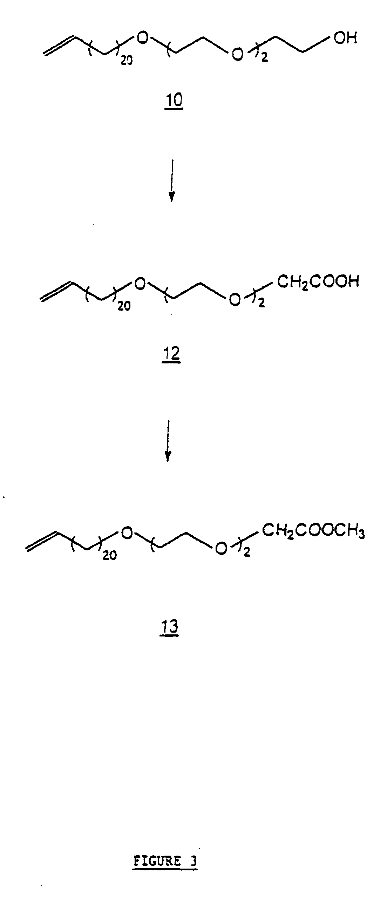

### 4) Préparation des esters insaturés (figure 3).

A partir de l'alcool insaturé 10 obtenu précédemment, l'acide et l'ester correspondants ont été préparés comme suit.

### • Préparation de l'acide 12

Dans un ballon tricol de 100 ml, l'alcool insaturé 10 (3 g; 6,6 mmol) est mis en suspension dans 10 ml d'acétone. A cette suspension est ajouté 80 ml de réactif de Jones 2M (BOWDEN *et al., J*. *Chem. Soc*., 1946, *39*). La suspension est portée au reflux pendant 2 heures. Après retour à température ambiante, l'acétone est évaporée, le solide est filtré puis rincé 5 fois à l'eau et 3 fois à l'acétone refroidie à 0°C. Le solide est ensuite purifié par recristallisation dans un mélange THF/acétone (v/v: 9/1) pour donner le composé 12 sous la forme d'un solide blanc (2,9 g; rendement de 94%). Son analyse par infra-rouge et RMN du proton et du carbone 13 est la suivante.
IR (dispersion dans KBr) ν (cm⁻¹): 3370; 3080; 2917; 2849; 1707; 1643.
RMN ¹H (CDCl₃; δ (ppm)): 11,2 (s large; 1H); 6,0-5,7 (m; 1 H); 5,1-4,7 (m; 2 H); 4,1-4,0 (s; 2 H) 3,6-3,3 (m; 10 H); 2,2-1,9 (m; 2 H) et 1,7-1,0 (m; 36 H).
RMN ¹³C (CDCl₃; δ (ppm)): 172,1; 139,1; 114,0; 71,6-68,7 (5 CH₂); 63,5; 33,6-25,8 (19 CH₂).

### • Préparation de l'ester 13

Dans un ballon tricol de 100 ml, l'acide 12 (2,9 g; 6,4 mmol) est solubilisé dans du toluène anhydre (7 ml) sous atmosphère inerte à 0°C. Du chlorure d'oxalyle (1,22 g; 9,6 mmol; 1,5 éq.) est introduit goutte à goutte puis le mélange est agité à température ambiante pendant deux heures. L'excès de réactif et le solvant sont ensuite évaporés sous vide. Le chlorure d'acyle est stocké temporairement sous argon. Du méthanol (6 ml; 128 mmol; 20 éq.), préalablement distillé sur chlorure de calcium, est ajouté lentement. Le milieu réactionnel est ensuite porté au reflux pendant 18 h avant d'être ramené à température ambiante, puis l'excès de méthanol est évaporé. Le milieu réactionnel est alors extrait trois fois à l'éther diéthylique. Les phases éthérées sont rassemblées, lavées avec une solution d'acide chlorhydrique à 10%, à l'eau et avec une solution saturée en NaHCO₃. La phase éthérée est ensuite lavée jusqu'à neutralité, séchée sur MgSO₄ et concentrée sous vide. Le composé 13 est obtenu sous la forme d'un solide blanc, puis est purifié par chromatographie sur silice (éluant : éther de pétrole/éther, 50/50 en volume). On obtient 300 mg d'un solide blanc (rendement de 10%). Son analyse par infra-rouge et RMN du proton et du carbone 13 est la suivante.
IR (dispersion dans KBr) ν (cm⁻¹): 3080; 2917; 2849; 1742; 1643.
RMN ¹H (CDCl₃; δ (ppm)): 6,0-5,7 (m; 1 H); 5,1-4,7 (m; 2 H); 4,1-4,0 (s; 2 H) 3,6-3,3 (m; 13 H); 2,2-1,9 (m; 2 H) et 1,7-1,0 (m; 36 H).
RMN ¹³C (CDCl₃; δ (ppm)): 171,8; 139,1; 114,0; 71,6-68,7 (5 CH₂); 63,6; 51,7; 33,6-25,8 (19 CH₂).

Il est bien entendu qu'en utilisant l'alcool 8 comprenant un seul motif éthylène glycol, les acides et les esters correspondants pourraient également être obtenus selon les mêmes protocoles que décrits ici à partir de l'alcool 10.

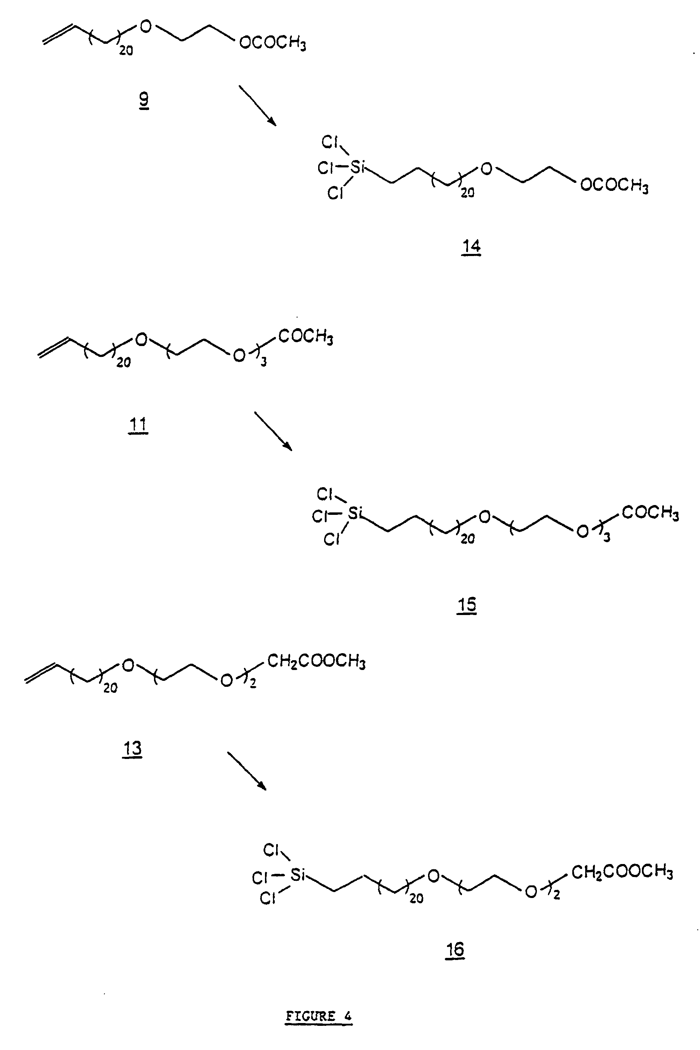

### 5) Silylation des précurseurs insaturés (figure 4).

Dans un schlenk sec, sous atmosphère inerte, l'ester 9 (150 mg; 0,33 mmol) est introduit. Du trichlorosilane fraîchement distillé (0,3 ml; 2,2 mmol; 6 éq.), du toluène anhydre (0,3 ml) ainsi qu'une goutte de catalyseur de Kärsted (PCO 72), commercialisé par ABCR (référence 68478-92-2), sont ajoutés. Le milieu réactionnel est alors porté à 40°C durant 2 h. Après retour à température ambiante, le toluène et l'excès de trichlorosilane sont évaporés sous pression réduite à l'aide d'une pompe à palette (pression de 0,5 mm de Hg). Le composé 14 est obtenu sous la forme d'un solide blanc et est stocké sous argon (rendement de 99%). Il s'agit d'un composé organosilicié de formule (I) telle que définie précédemment, dans laquelle X₁, X₂ et X₃ représentent des atomes de chlore, n est égal à 22, m est égal à 1, i est égal à 0, k est égal à 1 et B représente un groupement -COCH₃. L'analyse du composé 14 par RMN du proton et du carbone 13 est la suivante.
RMN ¹H (CDCl₃; δ (ppm)): 4,25-4,15 (m; 2H); 3,60-3,50 (t; 2 H); 3,45-3,35 (t; 2 H); 2,2-1,9 (s; 3 H) et 1,7-1,0 (m; 42 H).
RMN ¹³C (CDCl₃; δ (ppm)): 171,0; 71,5; 68,5; 63,7; 31,9-22,3 (21 CH₂); 21.0.

En partant du composé 11 obtenu ci-avant et en utilisant le même protocole, on obtient le composé organosilicié 15 correspondant. Il s'agit d'un composé organosilicié de formule (I) dans laquelle X₁, X₂ et X₃ représentent des atomes de chlore, n est égal à 22, m est égal à 1, i est égal à 0, k est égal à 3 et B représente un groupement -COCH₃. Son analyse par RMN du proton et du carbone 13 est la suivante. RMN ¹H (CDCl₃; δ (ppm)): 4,3-4,2 (t; 2H); 3,7-3,4 (m; 10 H); 3,4-3,3 (t; 2 H); 2,2-1,9 (s; 3 H) et 1,7-0,9 (m; 42 H). RMN ¹³C (CDCl₃; δ (ppm)): 171,2; 71,6-68,1 (6 CH₂); 63,6; 31,9-22,3 (21 CH₂); 21,0.

En partant du composé 13 obtenu ci-avant et en utilisant le même protocole, on obtient le composé organosilicié 16 correspondant. Il s'agit d'un composé de formule (I) dans laquelle X₁, X₂ et X₃ représentent des atomes de chlore, n est égal à 22, m est égal à 1, i est égal à 1, k est égal à 2 et B représente un groupement -COOCH₃. Son analyse par RMN du proton et du carbone 13 est la suivante. RMN ¹H (CDCl₃; δ (ppm)): 4,1-4,0 (s; 2 H); 3,6-3,3 (m; 13 H); 1,7-0,9 (m; 42 H). RMN ¹³C (CDCl₃; δ (ppm)): 171,0; 71,6-63,8 (6 CH₂); 51,7; 31,9-22,3 (21 CH₂).

Le tableau I ci-dessous récapitule la structure des composés organosiliciés de formule (I) obtenus dans le présent exemple.

**TABLEAU I**

| Composé organosilicié | X₁, X₂, X₃ | n | m | k | i | B |
|---|---|---|---|---|---|---|
| 14 | Cl | 22 | 1 | 1 | 0 | -COCH₃ |
| 15 | Cl | 22 | 1 | 3 | 0 | -COCH₃ |
| 16 | Cl | 22 | 1 | 2 | 1 | -COOCH₃ |

### EXEMPLE 2 : Synthèse de composés organosiliciés de formule (I) dans laquelle m = 0.

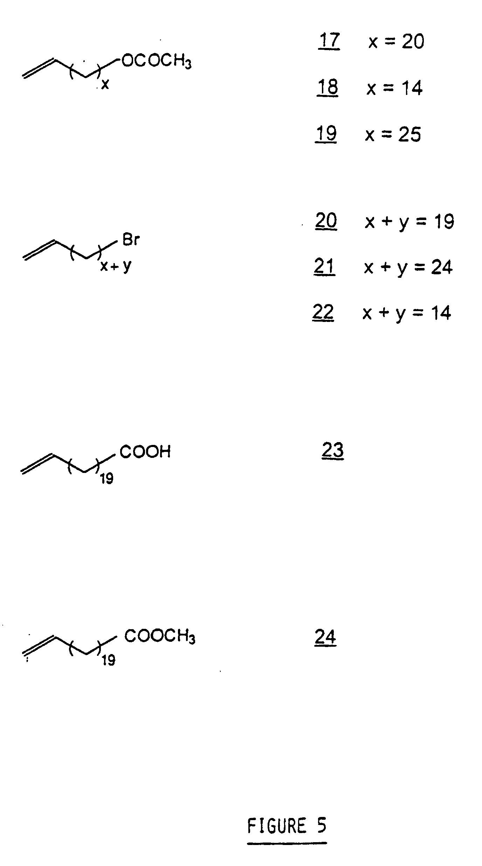

### 1) Synthèse de précurseurs insaturés (figure 5).

### 1-1 : précurseurs insaturés portant un groupement -OCOCH₃

### • Composé 17

Dans un ballon bicol de 100 ml, l'alcool insaturé 5 obtenu dans l'exemple 1 (0,9 g; 2,7 mmol) est mis en suspension dans du dichlorométhane (10 ml) et de la triéthylamine (0,6 ml; 5,4 mmol; 2 éq.) . Le milieu réactionnel est refroidi à 0°C, puis du chlorure d'acétyle (0,5 ml; 4 mmol; 1,5 éq.) est ajouté goutte à goutte à l'aide d'une seringue. Le milieu réactionnel est agité 15 minutes à 0°C, puis 1h30 à température ambiante. Le milieu réactionnel est ensuite hydrolysé, puis extrait trois fois à l'éther diéthylique. Les phases éthérées sont rassemblées, lavées avec une solution d'acide chlorhydrique à 10%, à l'eau, puis avec une solution saturée en NaHCO₃. La phase éthérée est ensuite lavée jusqu'à neutralité, séchée sur MgSO₄ et concentrée sous vide. Le composé 17 est obtenu sous la forme d'un solide blanc (0,9 g; rendement de 100%). Son analyse par infra-rouge et RMN du proton et du carbone 13 est la suivante.
IR (dispersion dans KBr) ν (cm⁻¹): 3079; 2919; 2850; 1740; 1641.
RMN ¹H (CDCl₃; δ (ppm)): 5,9-5,7 (m; 1 H); 5,1-4,7 (m; 2 H); 4,1-3,9 (t; 2H); 2,2-1,9 (m; 5 H) et 1,7-1,0 (m; 36 H).
RMN ¹³C (CDCl₃; δ (ppm)): 171,1; 139,3; 114,0; 64,7; 33,6-25,8 (19 CH₂); 21,0.

### • Composé 18

En utilisant le même protocole que pour l'obtention du composé 17, mais en partant d'un alcool insaturé comprenant 16 atomes de carbone, on obtient le composé 18 dont l'analyse par infra-rouge et RMN du proton et du carbone 13 est la suivante : IR (dispersion dans KBr) ν (cm⁻¹): 3080; 2923; 2853; 1742; 1643. RMN ¹H (CDCl₃; δ (ppm)): 5,9-5,7 (m; 1 H); 5,1-4,7 (m; 2 H); 4,1-4,0 (t; 2H); 2,1-1,9 (m; 5 H) et 1,7-1,1 (m; 24 H). RMN ¹³C (CDCl₃; δ (ppm)): 171,1; 139,3; 114,0; 64,8; 33,6-25,8 (13 CH₂); 21,2.

### • Composé 19

En utilisant le même protocole que pour l'obtention du composé 17, mais en partant d'un alcool insaturé comprenant 27 atomes de carbone, on obtient le composé 19 dont l'analyse par infra-rouge et RMN du proton et du carbone 13 est la suivante : IR (dispersion dans KBr) ν (cm⁻¹): 3080; 2923; 2853; 1742; 1643. RMN ¹H (CDCl₃; δ (ppm)): 5,9-5,7 (m; 1 H); 5,1-4,7 (m; 2 H); 4,1-4,0 (t; 2H); 2,1-1,9 (m; 5 H) et 1,7-1,1 (m; 46 H). RMN ¹³C (CDCl₃; δ (ppm)): 171,1; 139,3; 114,0; 64,8; 33,6-25,8 (24 CH₂); 21,1.

### 1-2 : précurseurs insaturés bromés

A partir de dérivés bromés insaturés de formule CH₂=CH-(CH₂)ₓBr, x étant compris entre 3 et 23, et en suivant le protocole d'obtention du composé 2 tel que décrit dans l'exemple 1, des organomagnésiens de formule CH₂=CH-(CH₂)ₓMgBr sont obtenus. Leur couplage avec un dérivé dibromé Br(CH₂)_{y}Br (x + y étant égal au minimum à 13 et au maximum à 33), dans les mêmes conditions que celles du couplage avec un bromoalcool conformément à l'exemple 1 pour l'obtention du composé 5, conduit aux dérivés bromés insaturés de formules 20, 21 et 22.

Le composé 20 est obtenu à l'aide d'un dérivé bromé insaturé dans lequel x est égal à 9 et d'un dérivé dibromé dans lequel y est égal à 10. Son analyse par infra-rouge et RMN du proton et du carbone 13 est la suivante : IR (dispersion dans KBr) ν (cm⁻¹): 3078; 2922; 2852; 1640. RMN ¹H (CDCl₃; δ (ppm)): 5,9-5,7 (m; 1 H); 5,1-4,9 (m; 2 H); 3,5 (t; 2 H); 2,2-1,8 (m; 4 H) et 1,6-1,2 (m; 32 H). RMN ¹³C (CDCl₃; δ (ppm)): 139,2; 114,1; 33,9-25,7 (19 CH₂).

Le composé 21 est obtenu à l'aide d'un dérivé bromé insaturé dans lequel x est égal à 14 et d'un dérivé dibromé dans lequel y est égal à 10. Son analyse par infra-rouge et RMN du proton et du carbone 13 est la suivante : IR (dispersion dans KBr) ν (cm⁻¹): 3080; 2920; 2851; 1642. RMN ¹H (CDCl₃; δ (ppm)): 6,0-5,7 (m; 1 H); 5,1-4,9 (m; 2 H); 3,5 (t; 2 H); 2,2-1,8 (m; 4 H) et 1,6-1,2 (m; 42 H). RMN ¹³C (CDCl₃; δ (ppm)): 138,8; 113,8; 33,9-25,7 (24 CH₂).

Le composé 22 est obtenu à l'aide d'un dérivé bromé insaturé dans lequel x est égal à 9 et d'un dérivé dibromé dans lequel y est égal à 5. Son analyse par infra-rouge et RMN du proton et du carbone 13 est la suivante : IR (dispersion dans KBr) ν (cm⁻¹): 3078; 2925; 2854; 1641. RMN ¹H (CDCl₃; δ (ppm)): 5,9-5,7 (m; 1 H); 5,1-4,9 (m; 2 H); 3,5 (t; 2 H); 2,2-1,8 (m; 4 H) et 1,6-1,2 (m; 22 H). RMN ¹³C (CDCl₃; δ (ppm)): 139,2; 114,1; 33,9-25,7 (14 CH₂).

### 1-3 : précurseurs insaturés portant un groupement acide carboxylique -COOH

Dans un ballon tricol de 250 ml, l'alcool insaturé 5 obtenu dans l'exemple 1 (13 g; 40 mmol) est mis en suspension dans 40 ml d'acétone. A cette suspension est ajouté 80 ml de réactif de Jones (2M). La suspension est portée au reflux pendant 2 heures. Après retour à température ambiante, l'acétone est évaporée, le solide est filtré puis rincé 5 fois à l'eau et 3 fois à l'acétone refroidie à 0°C. Le solide est ensuite purifié par recristallisation dans un mélange THF/acétone (v/v: 9/1) pour donner le composé 23 sous la forme d'un solide blanc (9,9 g; point de fusion de 73-74°C; rendement de 94%). Son analyse par infra-rouge et RMN du proton et du carbone 13 est la suivante: IR (dispersion dans KBr) ν (cm⁻¹): 3370; 3080; 2917; 2849; 1707; 1643. RMN ¹H (CDCl₃; δ (ppm)): 11,2 (s élargi; 1 H); 6,0-5,7 (m; 1 H); 5,1-4,9 (m; 2 H); 2,4 (t; 2 H); 2,2-2,0 (m; 2 H) et 1,9-1,1 (m; 34 H). RMN ¹³C (CDCl₃; δ (ppm)): 172,0; 139,3; 113,8; 33,6-25,8 (19 CH₂).

### 1-4 : précurseurs insaturés portant un groupement ester méthylique -COOCH₃

Dans un ballon tricol de 100 ml, l'acide 23 obtenu ci-dessus (4 g; 11,8 mmol) est solubilisé dans du toluène anhydre (20 ml) sous atmosphère inerte à 0°C. Du chlorure d'oxalyle (2 g; 17,6 mmol; 1,5 éq.) est introduit goutte à goutte, puis le mélange est agité à température ambiante pendant deux heures. L'excès de réactif et le solvant sont ensuite évaporés sous vide. Le chlorure d'acyle est stocké temporairement sous argon. Du méthanol (10 ml; 236 mmol; 20 éq.) préalablement distillé sur chlorure de calcium est ajouté lentement. Le milieu réactionnel est ensuite porté au reflux pendant 18 heures. Après retour du mélange réactionnel à température ambiante, l'excès de méthanol est évaporé. Le milieu réactionnel est alors extrait trois fois à l'éther diéthylique. Les phases éthérées sont rassemblées, lavées avec une solution d'acide chlorhydrique à 10%, à l'eau et avec une solution saturée en NaHCO₃. La phase éthérée est ensuite lavée jusqu'à neutralité, séchée sur MgSO₄ et concentrée sous vide. Le composé 24 est obtenu sous la forme d'un solide blanc (4 g; point de fusion de 50-51,5°C; rendement de 100%). Son analyse par infra-rouge et RMN du proton et du carbone 13 est la suivante : IR (dispersion dans KBr) ν (cm⁻¹): 3080; 2917; 2849; 1743; 1643. RMN ¹H (CDCl₃; δ (ppm)): 6,0-5,7 (m; 1 H); 5,1-4,9 (m; 2 H); 3,6 (t; 3 H); 2,4-2,3 (t; 2 H); 2,2-2,0 (m; 2 H) et 1,9-1,1 (m; 34 H). RMN ¹³C (CDCl₃; δ (ppm)): 172,3; 139,3; 114,0; 51,5; 33,6-25,8 (19 CH₂).

### 2) Silylation des précurseurs insaturés.

On procède selon le même mode opératoire que celui indiqué dans l'exemple 1. Les spectres RMN du proton et du carbone 13 des composés organosiliciés obtenus à partir des précurseurs insaturés 17, 18 et 19 sont les suivants :
- à partir du précurseur 17 : RMN ¹H (CDCl₃; δ (ppm)): 4,1-3,9 (t; 2H); 2,1 (s; 3 H); 1,7-0,9 (m; 42 H). RMN ¹³C (CDCl₃; δ (ppm)): 171,2; 64,7; 31,9-22,3 (21 CH₂); 21,0.
- à partir du précurseur 18 : RMN ¹H (CDCl₃; δ (ppm)): 4,1-3,9 (t; 2H); 2,1 (s; 3 H); 1,7-0,9 (m; 30 H). RMN ¹³C (CDCl₃; δ (ppm)): 171,2; 64,7; 31,9-22,3 (15 CH₂); 21.0.
- à partir du précurseur 19 : RMN ¹H (CDCl₃; δ (ppm)): 4,1-3,9 (t; 2H); 2,1 (s; 3 H); 1,7-0,9 (m; 52 H). RMN ¹³C (CDCl₃; δ (ppm)): 171,2; 64,7; 31,9-22,3 (26 CH₂); 21,0.

Le tableau II ci-dessous récapitule la structure des composés organosiliciés de formule (I) obtenus dans le présent exemple.

**TABLEAU II**

| Silylation du précurseur n° | X₁, X₂, X₃ | n | m | B |
|---|---|---|---|---|
| 17 | Cl | 22 | 0 | -OCOCH₃ |
| 18 | Cl | 16 | 0 | -OCOCH₃ |
| 19 | Cl | 27 | 0 | -OCOCH₃ |
| 24 | Cl | 21 | 0 | -COOCH₃ |

### EXEMPLE 3 : Silanisation d'un support solide à l'aide d'un composé organosilicié de formule (I) et obtention d'une monocouche autoassemblée organisée.

### 1) Silanisation du support solide.

Un disque de silicium, oxydé en surface, est utilisé comme substrat. Le disque est nettoyé selon la procédure suivante, afin d'éliminer les contaminants de sa surface et de l'hydrater :
- immersion dans un mélange sulfochromique fraîchement préparée (2,5 g de K₂Cr₂O₄; 2,5 ml d'eau distillé; 50 ml d'acide sulfurique) pendant 10 minutes,
- sous une hotte à flux laminaire équipée de filtres à poussières, le disque est plongé dans de l'eau permutée et soumis aux ultrasons durant 20 minutes. Ce processus est répété deux fois avec des durées de sonication de 5 et 2 minutes respectivement,
- sous une hotte à flux laminaire équipée de filtres à poussières, le disque est introduit dans le réacteur de silanisation pour être séché, sous atmosphère inerte et filtrée. Le réacteur est plongé pendant 45 minutes dans un bain d'huile à 100°C puis est retiré du bain d'huile et sa température ramenée à 18°C.

Le composé organosilicié 14 obtenu dans l'exemple 1, fraîchement préparé en quantité voulue, sous atmosphère inerte, est solubilisé dans une fraction d'un mélange C₆H₁₂/CCl₄/CHCl₃ (v/v/v: 80/12/8). Le composé organosilicié 14 en solution est ensuite prélevé à la seringue puis introduit dans un schlenck contenant le reste du mélange de solvant dont le volume total a été calculé pour obtenir une solution de silanisation de dilution adéquate (entre 1.10⁻⁵ et 1.10⁻² Mole/litre). Les solvants ont été préalablement séchés selon des procédures connues en elles-mêmes.

La solution de silanisation est introduite, avec une seringue, dans le réacteur et le disque de silicium reste immergé dans cette solution durant 16 h. Le disque silanisé est retiré du réacteur, puis éventuellement recuit, après avoir été lavé au chloroforme (« HPLC grade »), à une température comprise entre 50 et 120°C. Il est ensuite nettoyé aux ultrasons pendant 2 minutes, processus répété deux fois.

### 2) Caractérisation de la surface modifiée.

On a obtenu ci-dessus un support solide dont la surface est modifiée par le composé 14. Le contrôle du greffage des composés organosiliciés est réalisé en utilisant la spectroscopie infra-rouge et la spectroscopie Raman confocale.

### 3) Libération des hydroxyles de surface.

Si nécessaire, les hydroxyles de surface peuvent être libérés en utilisant le protocole suivant : le disque silanisé est immergé dans une solution de KOH (0,5 M) dans un mélange eau/éthanol (v/v:1/1) durant 20 minutes. Le disque est ensuite nettoyé aux ultrasons durant 5 minutes dans de l'eau déminéralisée. Ce processus est renouvelé une fois dans l'eau puis une deuxième fois dans le chloroforme.

### 4) Caractérisation de la surface après libération des hydroxyles.

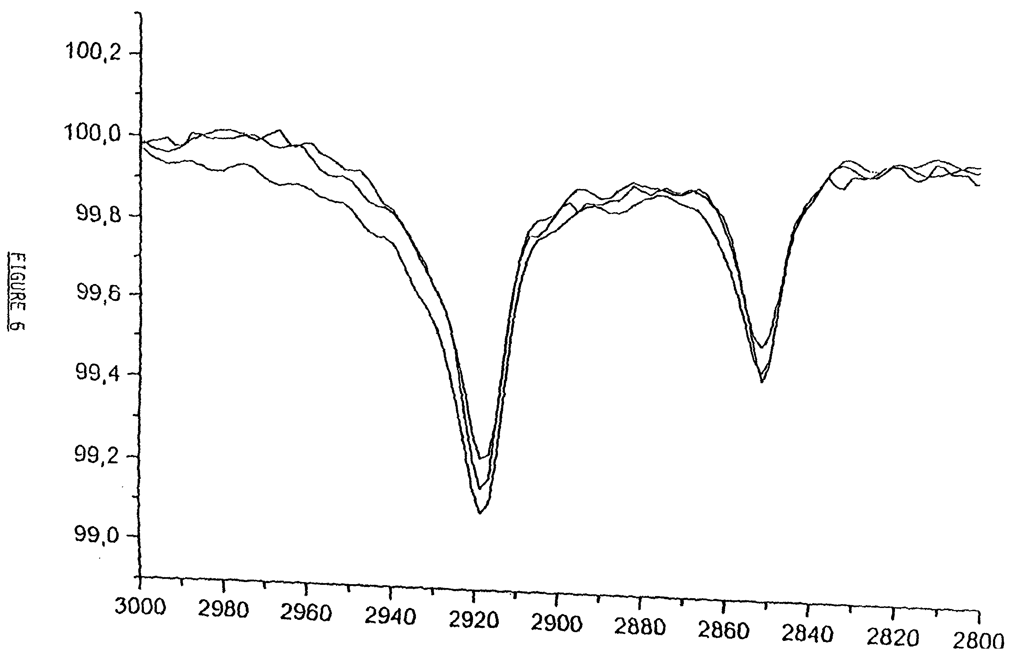

La figure 6 représente des spectres infra-rouge réalisés après trois expériences de greffage du composé 14 sur le support, selon le protocole de silanisation exposé ci-dessus et après saponification des esters de surface. La transmittance figure en ordonnées et la fréquence (cm⁻¹) en abscisses. On remarque que les trois spectres se superposent, avec des pics caractéristiques d'un système organisé à 2917 et à 2850 cm⁻¹. Ainsi, on peut en conclure que le greffage du composé organosilicié sur le support est reproductible et donne lieu à la formation d'une monocouche autoassemblée organisée.

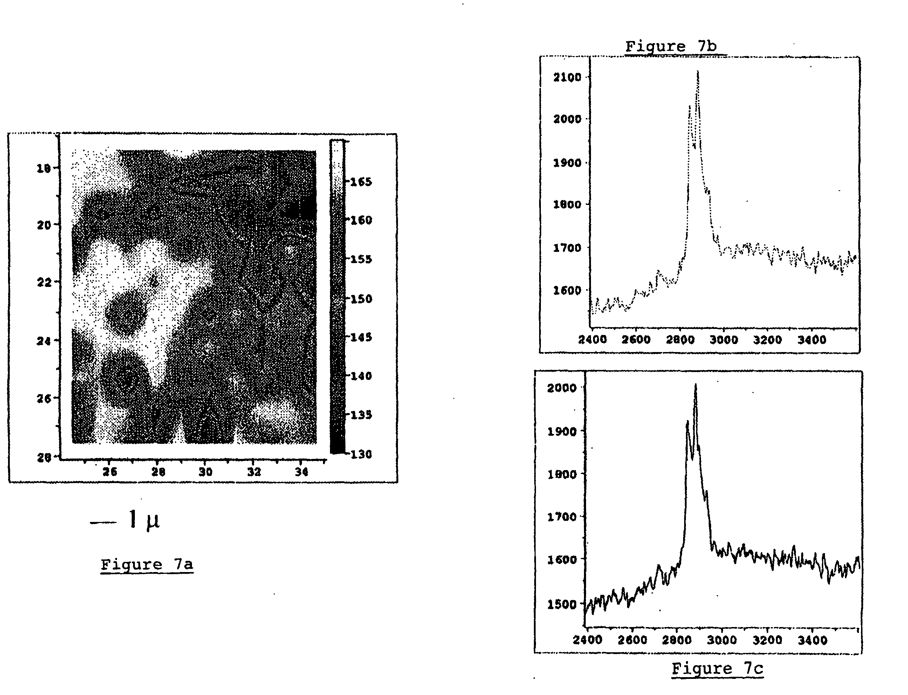

La figure 7 représente des études spectroscopiques Raman de la surface modifiée obtenue ci-dessus. La figure 7a est représentative de la densité de la surface du substrat greffé, les dimensions de la surface figurant en abscisses et en ordonnées (7 mm sur la figure correspondent à 1 µm sur le substrat) et l'échelle des densités étant graduée de 130 à 165 (unités arbitraires). Cette figure démontre l'homogénéité de la surface. Les figures 7b et 7c représentent les spectres Raman pris en deux points différents de la surface du substrat ; les ordonnées représentent des coups par seconde et la fréquence figure en abscisses.

Les spectres infra-rouge et Raman montrent donc sans ambiguïté l'homogénéité du film déposé sur le substrat ainsi que l'organisation des molécules sur la surface, caractéristique d'une monocouche autoassemblée organisée. Le spectre infra-rouge montre également que le greffage du film est parfaitement reproductible.

### EXEMPLE 4 : Autre exemple de silanisation d'un support solide à l'aide d'un composé organosilicié de formule (I).

Une lame de verre de microscope est utilisée comme substrat. La lame de verre est nettoyée par immersion dans une solution aqueuse d'Hellmanex® à 2% (commercialisée par Polylabo sous la référence 12240) pendant 2 h à 20°C, puis rinçage abondant à l'eau permutée. Sous une hotte à flux laminaire équipée de filtres à poussières, la lame de verre est introduite dans le réacteur de greffage pour être séchée, sous atmosphère inerte et filtrée. Le réacteur est plongé pendant 45 minutes dans un bain d'huile à 100°C, puis est retiré du bain d'huile et sa température est ramenée à 18°C.

La solution de silanisation, préparée à l'aide du composé organosilicié 14 comme indiqué dans l'exemple précédent, est introduite, avec une seringue, dans le réacteur et la lame de verre reste immergée dans cette solution durant 16 h. Le rinçage du substrat silanisé est effectué comme décrit dans l'exemple 3.

La caractérisation de la surface par spectroscopie infra-rouge et Raman démontrent là encore l'obtention d'une monocouche autoassemblée organisée sur le substrat, les mêmes résultats étant également obtenus avec d'autres agents organosiliciés de formule (I) que le composé 14.

### EXEMPLE 5: Synthèse directe d'acides nucléiques sur un support solide modifié par une monocouche autoassemblée organisée de composés organosiliciés de formule (I).

### 1) Préparation du support solide modifié par une monocouche autoassemblée organisée.

Le support solide utilisé est un substrat de silicium oxydé Si/SiO₂ sur lequel a été formée une monocouche autoassemblée organisée comprenant le composé organosilicié 14, selon le protocole indiqué dans l'exemple 3. On pourrait également utiliser tout autre composé organosilicié de formule (I), ainsi que tout mélange de composés organosiliciés comprenant au moins un composé organosilicié de formule (I).

Les extrémités des composés organosiliciés greffés sur le support sont converties en groupement hydroxyles par immersion du substrat dans une solution de KOH, comme indiqué dans l'exemple 3. On obtient ainsi un substrat comportant des fonctions hydroxyles de surface. Ce substrat est nettoyé dans l'acétonitrile et est soumis aux ultrasons pendant 1 minute avant d'être placé dans la cellule de synthèse.

### 2) Synthèse d'acides nucléiques sur ce support.

On utilise ici la chimie des phosphoramidites, sachant que d'autres chimies bien connues de l'Homme du métier pourraient être utilisées, telle que la chimie des H-phosphonates. Les nucléotides sont donc utilisés sous forme de β-cyanoéthyl-phosphoramidites, les amines des différentes bases azotées étant protégées, par exemple et de façon non limitative, par des groupements benzoyle, isobutyryle ou tertiobutylphénoxyacétique. Les nucléotides sont protégés en position 5' par un groupement diméthoxytrityle ; on pourrait toutefois également utiliser des groupements photolabiles (tels que le MeNPOC décrit par PEASE *et al*. dans *Proc. Natl*. *Acad. Sci*. USA, 1994, *91*, 5022-5026), qui pourraient être éliminés par illumination UV.

La synthèse d'acides nucléiques est, avec la chimie des phosphoramidites utilisée, réalisée dans le sens 3' → 5'. Les différentes étapes de greffage d'un nucléotide sont les suivantes, l'ensemble de ces étapes constituant un cycle :
1. détritylation (25 secondes) : acide trichloroacétique à 3% dans le dichlorométhane ;
2. lavage : acétonitrile anhydre ;
3. couplage (1 minute) : introduction d'un mélange d'amidite 0,1 M et d'un activateur (tétrazole) 0,45 M ;
4. capping (15 secondes) : introduction d'un mélange de (i) anhydride acétique/2,6-lutidine/acétonitrile (10 ml/10 ml/80 ml) et de (ii) 1-méthyl-imidazole à 10% dans le diméthylformamide (10 ml/90 ml) ;
5. oxydation (15 secondes) : introduction d'un mélange iode/pyridine/tétrahydrofurane/eau (0,508 mg/1,6 ml/15,2 ml/3,2 ml) ;
6. capping (15 secondes).

Il est bien entendu que, dans le cycle donné ci-dessus, les solutions de détritylation, de lavage, de couplage, de capping et d'oxydation ne sont pas données à titre limitatif.

On peut effectuer autant de cycles que désiré, suivant la longueur de l'acide nucléique que l'on souhaite obtenir. Une fois le nombre final de cycles atteint, les bases azotées sont déprotégées et l'acide nucléique est déprotégé par un traitement à l'ammoniaque à 30-33% pendant une nuit à température ambiante, de manière à cliver les groupements protecteurs répartis sur le squelette et les bases azotées. Eventuellement, si un bras espaceur labile à l'ammoniaque a été introduit entre une partie des acides nucléiques et la surface silanisée, cette fraction des acides nucléiques sera clivée du support.

Selon la nature des groupements protecteurs utilisés, on peut utiliser d'autres traitements de déprotection, par exemple à l'aide de carbonate de potassium.

Le substrat est lavé à l'eau puis au tampon TRIS (solution de TRIS HCl 10 mM ajustée à pH 7,1 par ajout de solution de TRIS base 10 mM, la solution finale ayant une concentration en NaCl de 50 mM) avant de pouvoir être utilisé pour une hybridation.

### EXEMPLE 6 : Expériences d'hybridation réalisées avec des oligonucléotides synthétisés par voie directe sur un support solide d'après le procédé selon la présente invention.

On utilise un support solide modifié par une monocouche autoassemblée organisée tel que décrit dans l'exemple 5 (silicium oxydé Si/SiO₂ sur lequel est formée une monocouche de composés organosiliciés 14, les fonctions de surface ayant été converties en groupement hydroxyles). Un bras espaceur de 10 nucléotides T est greffé sur ce support modifié avant la synthèse oligonucléotidique.

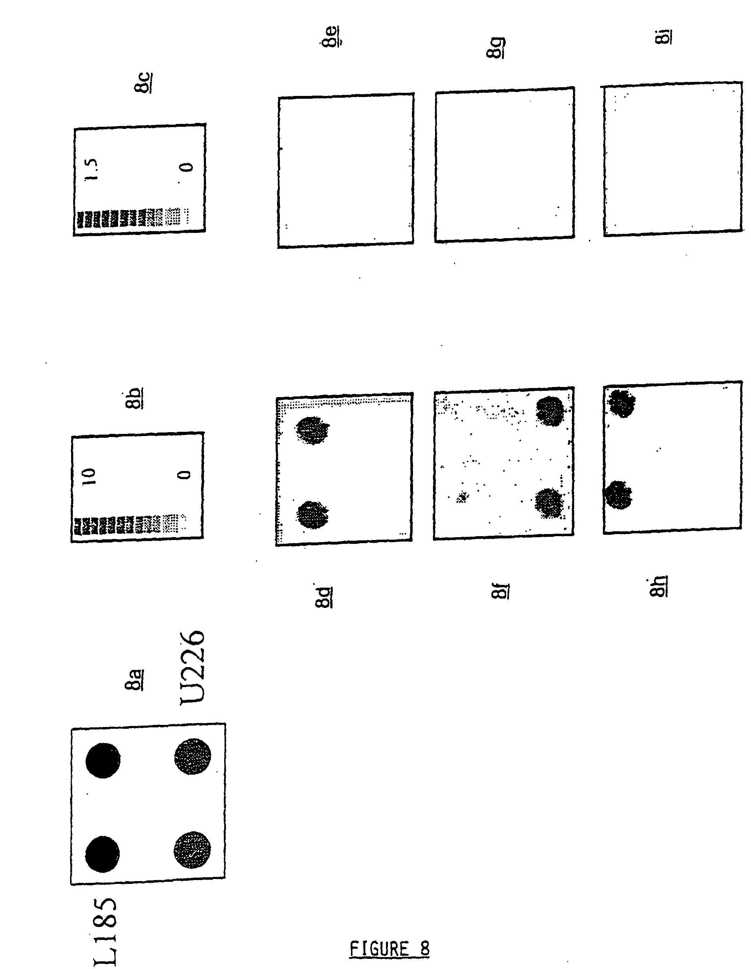

En quatre points de ce support sont effectuées des synthèses d'oligonucléotides appelés L185 et U226 (séquences de 25 nucléotides), comme illustré sur la figure 8a. Les figures 8b et 8c indiquent, sur une échelle de 0 à 10 pour la figure 8b (tenant lieu d'échelle pour les figures 8d, 8f et 8h) et sur une échelle de 0 à 1,5 pour la figure 8c (tenant lieu d'échelle pour les figures 8e, 8g et 8i), les intensités de fluorescence relevées sur le support.

La figure 8d représente l'intensité de fluorescence relevée après avoir mis en contact le support avec une solution d'un oligonucléotide complémentaire du L185 comprenant des marqueurs de fluorescence, après avoir lavé le support. L'hybridation est effectuée à 46°C pendant une nuit en présence d'un tampon TRIS pH 7,1 comprenant 50 mM de NaCl. La concentration de l'oligonucléotide L185 dans le TRIS est de 0,01 mg/ml. Chaque brin complémentaire de L 185 porte un groupement fluorescent Cy3 à l'extrémité 5'. On observe bien une hybridation de la séquence complémentaire avec la séquence L185. La figure 8e correspond au support après dénaturation : la séquence complémentaire n'est plus détectée.

La figure 8f représente l'intensité de fluorescence relevée après avoir mis en contact le support avec une solution d'un oligonucléotide complémentaire du U226 comprenant des marqueurs de fluorescence, après avoir lavé le support, dans les mêmes conditions qu'indiquées ci-dessus en rapport avec le brin L185. On observe là encore une hybridation de la séquence complémentaire avec la séquence U226. La figure 8g correspond au support après dénaturation : la séquence complémentaire n'est plus détectée.

La figure 8h représente l'intensité de fluorescence relevée après avoir de nouveau mis en contact le support avec une solution d'un oligonucléotide complémentaire du L185 comprenant des marqueurs de fluorescence, après avoir lavé le support. L'hybridation de la séquence complémentaire avec la séquence L185 se produit de nouveau. La figure 8i montre l'absence de la séquence complémentaire après dénaturation.

Ces expériences montrent que des acides nucléiques synthétisés par voie directe sur un support solide modifié par une monocouche autoassemblée organisée de composés organosiliciés de formule (I), synthèse effectuée conformément au procédé selon la présente invention, peuvent donner lieu à des réactions d'hybridation sélective avec des séquences complémentaires, le support étant réutilisable au cours de plusieurs cycles successifs d'hybridation et de dénaturation.

### EXEMPLE 7 : Immobilisation d'acides nucléiques présynthétisés sur un support solide modifié par une monocouche autoassemblée organisée de composés organosiliciés de formule (I).

### 1) Préparation du support solide modifié par une monocouche autoassemblée organisée.

On procède comme dans l'exemple 5, à la différence près que la monocouche autoassemblée organisée comprend le composé organosilicié de formule 16 préparé selon le protocole indiqué dans l'exemple 1. Il est bien entendu que l'on pourrait utiliser tout autre composé organosilicié de formule (I) (ou un mélange de composés organosiliciés comprenant au moins un composé organosilicié de formule (I)), ledit composé de formule (I) portant une fonction acide carboxylique protégée. Les extrémités des composés organosiliciés sont converties en groupement acides carboxyliques par hydrolyse des esters (par exemple sous l'action du triiodométhylsilane ou de l'acide chlorhydrique concentré).

### 2) Immobilisation d'acides nucléiques sur ce support.

Les fonctions acides carboxyliques du support sont activées par une solution de N-hydroxysuccinimide. Le support ainsi activé est mis en contact avec une solution de l'acide nucléique à immobiliser (1 µg/µl dans un mélange acétonitrile/eau 9/1 en volume, en présence de triéthylamine), cet acide nucléique portant un bras espaceur fonctionnalisé par une fonction amine. La réaction est effectuée à température ambiante, éventuellement jusqu'au séchage de la solution. Le support est ensuite lavé avec un tampon TRIS pH 7,1. Cette opération permet le blocage des fonctions acides qui n'auraient pas réagi par la réaction avec une amine (réaction de capping).

## Revendications

1. Utilisation, pour la synthèse ou l'immobilisation d'acides nucléiques, d'un support solide modifié par une monocouche autoassemblée organisée comprenant au moins un composé organosilicié répondant à la formule (I) : 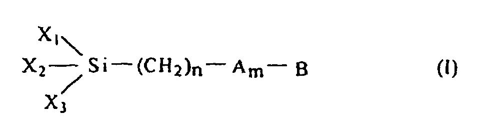 dans laquelle :
- n est compris entre 15 et 35,
- m est égal à 0 ou à 1,
- X₁, X₂ et X₃, qui peuvent être identiques ou différents entre eux, sont sélectionnés dans le groupe constitué par les groupes alkyles saturés en C₁ à C₆, linéaires ou ramifiés, et les groupements hydrolysables, au moins l'un de X₁, X₂ ou X₃ représentant un groupement hydrolysable,
- A représente le groupement -O-(CH₂-CH₂-O)ₖ-(CH₂)ᵢ- dans lequel k est compris entre 0 et 100 et i représente un nombre entier supérieur ou égal à 0,
- dans le cas où m = 0, alors B représente un groupement -OCOR, -OR, -COOR ou un atome d'halogène, R représentant un atome d'hydrogène ou un groupe alkyle en C₁ à C₆, linéaire ou ramifié,
- dans le cas où m = 1 :
. si k = 0 et i = 0, alors B représente un groupement R₁,
. si k = 0 et i ≥ 1, alors B représente un groupement -OR₁, -OCOR₁, -NR₁R₂, -COOR₁, -CONR₁R₂, -SR₁ ou un atome d'halogène,
. si k ≥ 1 et i = 0, alors B représente un groupement -R₁, -COR₁, -COOR₁ ou -CONR₁R₂,
. si k ≥ 1 et i ≥ 1, alors B représente un groupement -OR₁, -OCOR₁, -NR₁R₂, -COOR₁, -CONR₁R₂, -SR₁ ou un atome d'halogène,
. R₁ et R₂, qui peuvent être identiques ou différents, représentent un atome d'hydrogène, une chaîne hydrocarbonée éventuellement substituée, saturée ou insaturée et linéaire ou ramifiée, comprenant de 1 à 24 atomes de carbone, ou un groupement aromatique.

2. Utilisation selon la revendication 1, **caractérisée en ce que** n est compris entre 20 et 25.

3. Utilisation selon la revendication 1 ou la revendication 2, **caractérisée en ce que** k est compris entre 0 et 5.

4. Utilisation selon l'une quelconque des revendications précédentes, **caractérisée en ce que** ledit support solide est sélectionné dans le groupe constitué par les verres, les céramiques, les métaux et les métalloïdes.

5. Utilisation selon l'une quelconque des revendications précédentes, **caractérisée en ce que** ledit groupement hydrolysable est sélectionné dans le groupe constitué par les atomes d'halogène, le groupement -N(CH₃)₂ et les groupements -OR', R' étant un groupe alkyle saturé en C₁ à C₆, linéaire ou ramifié.

6. Utilisation selon l'une quelconque des revendications précédentes, **caractérisée en ce que** X₁, X₂ et X₃ représentent des atomes de chlore, n est égal à 22, m est égal à 1, i est égal à 0, k est égal à 1 ou à 3 et B représente un groupement -COCH₃.

7. Utilisation selon l'une quelconque des revendications 1 à 5, **caractérisée en ce que** X₁, X₂ et X₃ représentent des atomes de chlore, n est égal à 22, m est égal à 1, i est égal à 1, k est égal à 2 et B représente un groupement -COOCH₃.

8. Utilisation selon l'une quelconque des revendications 1 à 5, **caractérisée en ce que** X₁, X₂ et X₃ représentent des atomes de chlore, n est égal à 16, 22 ou 27, m est égal à 0 et B représente un groupement -OCOCH₃.

9. Utilisation selon l'une quelconque des revendications 1 à 5, **caractérisée en ce que** X₁, X₂ et X₃ représentent des atomes de chlore, n est égal à 21, m est égal à 0 et B représente un groupement -COOCH₃.

10. Utilisation selon l'une quelconque des revendications précédentes pour la préparation de puces à ADN.

11. Procédé de synthèse d'acides nucléiques sur un support solide, **caractérisé en ce que** ledit support est modifié par une monocouche autoassemblée organisée comprenant au moins un composé organosilicié répondant à la formule (I) telle que définie dans l'une quelconque des revendications 1 à 9, et **en ce qu'**il comprend les étapes suivantes :
a) préparation d'un support solide modifié par une monocouche autoassemblée organisée comprenant au moins un composé organosilicié répondant à la formule (I) telle que définie dans l'une quelconque des revendications 1 à 9, dans lequel lesdits composés organosiliciés présentent à leur extrémité une fonction hydroxyle ou amine protégée ;
b) éventuellement, déprotection de la fonction hydroxyle ou amine ;
c) couplage covalent, de façon localisée, d'un nucléotide sur le support solide modifié obtenu à l'étape a) ou b) ;
d) réitération de l'étape c) au moins une fois, avec des nucléotides identiques ou différents.

12. Procédé selon la revendication 11, **caractérisé en ce que** l'étape a) est réalisée par les étapes suivantes :
i) élimination des contaminants du support solide et hydratation et/ou hydroxylation de sa surface,
j) introduction dans un mélange d'au moins deux solvants comprenant au moins un solvant hydrocarboné non polaire, sous atmosphère inerte, d'un composé organosilicié de formule générale (I) telle que définie dans l'une quelconque des revendications 1 à 9, ledit composé présentant à une extrémité une fonction hydroxyle ou amine protégée,
k) silanisation du support obtenu à l'étape i) par immersion dans la solution préparée à l'étape j),
l) éventuellement, recuit du support silanisé obtenu à l'étape k), portant la monocouche autoassemblée, à une température comprise entre 50 et 120°C, pour une durée de 5 minutes à une nuit, et
m) rinçage du support modifié obtenu à l'étape k) ou l) à l'aide d'un solvant.

13. Procédé selon la revendication 11 ou la revendication 12, **caractérisé en ce qu'**une étape de greffage, à l'extrémité desdits composés organosiliciés, de bras espaceurs portant des fonctions hydroxyles ou amines terminales est effectuée après l'étape a) ou b) et avant l'étape c).

14. Procédé selon l'une quelconque des revendications 11 à 13, **caractérisé en ce que** les nucléotides couplés à l'étape c) sont protégés et **en ce que** l'étape d) est suivie d'une étape de déprotection de l'acide nucléique synthétisé.

15. Procédé d'immobilisation d'acides nucléiques sur un support solide, **caractérisé en ce que** ledit support est modifié par une monocouche autoassemblée organisée comprenant au moins un composés organosiliciés répondant à la formule (I) telle que définie dans l'une quelconque des revendications 1 à 9, et **en ce qu'**il comprend les étapes suivantes :
a) préparation d'un support solide modifié par une monocouche autoassemblée organisée comprenant au moins un composés organosiliciés répondant à la formule (I) telle que définie dans l'une quelconque des revendications 1 à 9, dans lequel lesdits composés organosiliciés présentent à leur extrémité une fonction acide carboxylique ou amine protégée ;
b) éventuellement, déprotection de la fonction acide carboxylique ou amine ;
c) éventuellement, dans le cas où le support solide modifié porte des fonctions acides carboxyliques terminales, activation de ces fonctions ;
d) mise en contact du support solide modifié obtenu à l'étape a), b) ou c) avec une ou plusieurs solutions appliquées localement, dans un ou plusieurs solvants polaires, du(des) acide(s) nucléique(s) à immobiliser, lesdits acides nucléiques portant l'une de leurs extrémités un bras espaceur fonctionnalisé soit par une fonction amine, dans le cas où le support solide modifié porte des fonctions acides carboxyliques terminales éventuellement protégées, soit par une fonction acide carboxylique activée, dans le cas où le support solide modifié porte des fonctions amines terminales éventuellement protégées ; et
e) lavage du support solide sur lequel sont immobilisés lesdits acides nucléiques.

16. Procédé selon la revendication 15, **caractérisé en ce que** l'étape a) est réalisée par les étapes suivantes :
i) élimination des contaminants du support solide et hydratation et/ou hydroxylation de sa surface,
j) introduction dans un mélange d'au moins deux solvants comprenant au moins un solvant hydrocarboné non polaire, sous atmosphère inerte, d'un composé organosilicié de formule générale (I) telle que définie dans l'une quelconque des revendications 1 à 9, ledit composé présentant à une extrémité une fonction acide carboxylique ou amine protégée,
k) silanisation du support obtenu à l'étape i) par immersion dans la solution préparée à l'étape j), et
l) éventuellement, recuit du support silanisé obtenu à l'étape k), portant la monocouche autoassemblée, à une température comprise entre 50 et 120°C, pour une durée de 5 minutes à une nuit, et
m) rinçage du support modifié obtenu à l'étape k) ou l) à l'aide d'un solvant.

17. Procédé selon la revendication 15 ou la revendication 16, **caractérisé en ce qu'**une étape de greffage, à l'extrémité desdits composés organosiliciés, de bras espaceurs portant des fonctions acides carboxyliques ou amines terminales est effectuée après l'étape a) ou b) et avant l'étape c).

18. Puce à ADN, **caractérisée en ce qu'**elle est obtenue par le procédé selon l'une quelconque des revendications 15 à 17, dans lequel lesdits acides nucléiques sont des ADN.

## Patentansprüche

1. Verwendung, zur Synthese oder Immobilisierung von Nucleinsäuren, eines festen Trägers, der durch eine selbstorganisierte Monoschicht modifiziert ist, die wenigstens eine siliciumorganische Verbindung der Formel (I) umfaßt: 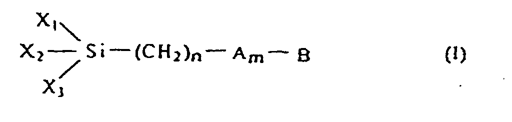 wobei
n zwischen 15 und 35 liegt,
m gleich 0 oder 1 ist,
X₁, X₂ und X₃, die gleich oder voneinander verschieden sein können, ausgewählt sind aus der Gruppe bestehend aus unverzweigten oder verzweigten gesättigten C₁-C₆-Alkylgruppen und hydrolysierbaren Gruppen, wobei wenigstens eines von X₁, X₂ oder X₃ eine hydrolysierbare Gruppe darstellt,
A die Gruppe -O-(CH₂-CH₂-O)ₖ-(CH₂)ᵢ- darstellt, worin k zwischen 0 und 100 liegt und i eine ganze Zahl höher oder gleich 0 ist,
falls m = 0, B eine Gruppe -OCOR, -OR, -COOR oder ein Halogenatom darstellt, wobei R ein Wasserstoffatom oder eine unverzweigte oder verzweigte C₁-C₆-Alkylgruppe darstellt,
falls m = 1:
wenn k = 0 und i = 0, B eine Gruppe R₁ darstellt,
wenn k = 1 und i ≥ 1, B eine Gruppe -OR₁, -OCOR₁, -NR₁R₂, -COOR₁, -CONR₁R₂, -SR₁ oder ein Halogenatom darstellt,
wenn k ≥ 1 und i = 0, B eine Gruppe -R₁, -COR₁, -COOR₁ oder -CONR₁R₂ darstellt,
wenn k ≥ 1 und i ≥ 1, B eine Gruppe -OR₁, -OCOR₁, -NR₁R₂, -COOR₁, -CONR₁R₂, -SR₁ oder ein Halogenatom darstellt,
R₁ und R₂, die gleich oder verschieden sein können, ein Wasserstoffatom, eine gegebenenfalls substituierte gesättigte oder ungesättigte und unverzweigte oder verzweigte Kohlenwasserstoffkette mit 1 bis 24 Kohlenstoffatomen oder eine aromatische Gruppe darstellen.

2. Verwendung nach Anspruch 1, **dadurch gekennzeichnet, dass** n zwischen 20 und 25 liegt.

3. Verwendung nach Anspruch 1 oder Anspruch 2, **dadurch gekennzeichnet, dass** k zwischen 0 und 5 liegt.

4. Verwendung nach irgendeinem der vorhergehenden Ansprüche, **dadurch gekennzeichnet, dass** der feste Träger ausgewählt ist aus der Gruppe bestehend aus Glasmaterialien, Keramikmaterialien, Metallen und Metalloiden.

5. Verwendung nach irgendeinem der vorhergehenden Ansprüche, **dadurch gekennzeichnet, dass** die hydrolysierbare Gruppe ausgewählt ist aus der Gruppe bestehend aus Halogenatomen, der Gruppe -N(CH₃)₂ und der Gruppe -OR', wobei R' eine unverzweigte oder verzweigte gesättigte C₁-C₆-Alkylgruppe ist.

6. Verwendung nach irgendeinem der vorhergehenden Ansprüche, **dadurch gekennzeichnet, dass** X₁, X₂ und X₃ Chloratome darstellen, n gleich 22 ist, m gleich 1 ist, i gleich 0 ist, k gleich 1 oder 3 ist und B eine Gruppe -COCH₃ darstellt.

7. Verwendung nach irgendeinem der Ansprüche 1 bis 5, **dadurch gekennzeichnet, dass** X₁, X₂ und X₃ Chloratome darstellen, n gleich 22 ist, m gleich 1 ist, i gleich 1 ist, k gleich 2 ist und B eine Gruppe -COOCH₃ darstellt.

8. Verwendung nach irgendeinem der Ansprüche 1 bis 5, **dadurch gekennzeichnet, dass** X₁, X₂ und X₃ Chloratome darstellen, n gleich 16, 22 oder 27 ist, m gleich 0 ist und B eine Gruppe -OCOCH₃ darstellt.

9. Verwendung nach irgendeinem der Ansprüche 1 bis 5, **dadurch gekennzeichnet, dass** X₁, X₂ und X₃ Chloratome darstellen, n gleich 21 ist, m gleich 0 ist und B eine Gruppe -COOCH₃ darstellt.

10. Verwendung nach irgendeinem der vorhergehenden Ansprüche zur Herstellung von DNA-Chips.

11. Verfahren zur Synthese von Nucleinsäuren auf einem festen Träger, **dadurch gekennzeichnet, dass** der Träger durch eine selbstorganisierte Monoschicht modifiziert ist, die wenigstens eine siliciumorganische Verbindung der Formel (I) umfaßt, wie sie in irgendeinem der Ansprüche 1 bis 9 definiert ist, und dadurch, dass es die folgenden Schritte umfaßt:
a) Herstellung eines festen Trägers, der durch eine selbstorganisierte Monoschicht modifiziert ist, die wenigstens eine siliciumorganische Verbindung der Formel (I) umfaßt, wie sie in irgendeinem der Ansprüche 1 bis 9 definiert ist, wobei die siliciumorganischen Verbindungen an ihrem Ende eine geschützte Hydroxy- oder Aminfunktion aufweisen;
b) gegebenenfalls Entschützen der Hydroxy- oder Aminfunktion;
c) lokalisierte kovalente Kupplung eines Nucleotids an den in den Schritten a) oder b) erhaltenen modifizierten festen Träger;
d) wenigstens einmaliges Wiederholen von Schritt c) mit gleichen oder anderen Nucleotiden.

12. Verfahren nach Anspruch 11, **dadurch gekennzeichnet, dass** Schritt a) mit den folgenden Schritten durchgeführt wird:
i) Eliminierung der Verunreinigungen von dem festen Träger und Hydratation und/oder Hydroxylierung seiner Oberfläche,
j) Einbringen in eine Mischung aus wenigstens zwei Lösungsmitteln, die wenigstens ein unpolares Lösungsmittel auf Kohlenwasserstoffbasis umfassen, unter Inertatmosphäre, einer siliciumorganischen Verbindung der allgemeinen Formel (I), wie sie in irgendeinem der Ansprüche 1 bis 9 definiert ist, wobei die Verbindung an einem Ende eine geschützte Hydroxy- oder Aminfunktion aufweist,
k) Silanisierung des in Schritt i) erhaltenen Trägers durch Eintauchen in die in Schritt j) hergestellte Lösung,
l) gegebenenfalls Tempern des in Schritt k) erhaltenen silanisierten Trägers, der die selbstorganisierte Monoschicht trägt, bei einer Temperatur zwischen 50 und 120°C für eine Dauer von 5 Minuten bis zu einer Nacht, und
m) Spülen des in Schritt k) oder l) erhaltenen modifizierten Trägers mit einem Lösungsmittel.

13. Verfahren nach Anspruch 11 oder Anspruch 12, **dadurch gekennzeichnet, dass** nach Schritt a) oder b) und vor Schritt c) ein Schritt des Aufpfropfens von Spacer-Armen, die endständige Hydroxy- oder Aminfunktionen tragen, an das Ende der siliciumorganischen Verbindungen durchgeführt wird.

14. Verfahren nach irgendeinem der Ansprüche 11 bis 13, **dadurch gekennzeichnet, dass** die in Schritt c) gekuppelten Nucleotide geschützt sind, und dadurch, dass auf Schritt d) ein Schritt zum Entschützen der synthetisierten Nucleinsäure folgt.

15. Verfahren zur Immobilisierung von Nucleinsäuren auf einem festen Träger, **dadurch gekennzeichnet, dass** der Träger durch eine selbstorganisierte Monoschicht modifiziert ist, die wenigstens eine siliciumorganische Verbindung der Formel (I) umfaßt, wie sie in irgendeinem der Ansprüche 1 bis 9 definiert ist, und dadurch, dass es die folgenden Schritte umfaßt:
a) Herstellung eines festen Trägers, der durch eine selbstorganisierte Monoschicht modifiziert ist, die wenigstens eine siliciumorganische Verbindung der Formel (I) umfaßt, wie sie in irgendeinem der Ansprüche 1 bis 9 definiert ist, wobei die siliciumorganischen Verbindungen an ihrem Ende eine geschützte Carbonsäure- oder Aminfunktion aufweisen;
b) gegebenenfalls Entschützen der Carbonsäure- oder Aminfunktion;
c) gegebenenfalls, wenn der modifizierte feste Träger endständige Carbonsäurefunktionen trägt, Aktivierung dieser Funktionen;
d) In-Kontakt-bringen des in Schritt a), b) oder c) erhaltenen modifizierten festen Trägers mit ein oder mehreren Lösungen, lokal aufgebracht, der zu immobilisierenden Nucleinsäure(n) in ein oder mehreren polaren Lösungsmitteln, wobei die Nucleinsäuren an einem ihrer Enden einen Spacer-Arm tragen, der entweder mit einer Aminfunktion funktionalisiert ist, wenn der modifizierte feste Träger gegebenenfalls geschützte endständige Carbonsäurefunktionen trägt, oder mit einer aktivierten Carbonsäurefunktion, wenn der modifizierte feste Träger gegebenenfalls geschützte endständige Aminfunktionen trägt; und
e) Waschen des festen Trägers, auf dem die Nucleinsäuren immobilisiert sind.

16. Verfahren nach Anspruch 15, **dadurch gekennzeichnet, dass** Schritt a) mit den folgenden Schritten durchgeführt wird:
i) Eliminierung der Verunreinigungen von dem festen Träger und Hydratation und/oder Hydroxylierung seiner Oberfläche,
j) Einbringen in eine Mischung aus wenigstens zwei Lösungsmitteln, die wenigstens ein unpolares Lösungsmittel auf Kohlenwasserstoffbasis umfassen, unter Inertatmosphäre, einer siliciumorganischen Verbindung der allgemeinen Formel (I), wie sie in irgendeinem der Ansprüche 1 bis 9 definiert ist, wobei die Verbindung an einem Ende eine geschützte Carbonsäure- oder Aminfunktion aufweist,
k) Silanisierung des in Schritt i) erhaltenen Trägers durch Eintauchen in die in Schritt j) hergestellte Lösung, und
l) gegebenenfalls Tempern des in Schritt k) erhaltenen silanisierten Trägers, der die selbstorganisierte Monoschicht trägt, bei einer Temperatur zwischen 50 und 120°C für eine Dauer von 5 Minuten bis zu einer Nacht, und
m) Spülen des in Schritt k) oder l) erhaltenen modifizierten Trägers mit einem Lösungsmittel.

17. Verfahren nach Anspruch 15 oder Anspruch 16, **dadurch gekennzeichnet, dass** nach Schritt a) oder b) und vor Schritt c) ein Schritt des Aufpfropfens von Spacer-Armen, die endständige Carbonsäure- oder Aminfunktionen tragen, an das Ende der siliciumorganischen Verbindungen durchgeführt wird.

18. DNA-Chip, **dadurch gekennzeichnet, dass** er nach dem Verfahren nach irgendeinem der Ansprüche 15 bis 17 erhalten wird, wobei die Nucleinsäuren DNAs sind.

## Claims

1. Use, to synthesise or immobilise nucleic acids, of a solid support modified by an organised, self-assembled mono-layer comprising at least one organo-silicon compound corresponding to Formula (I): 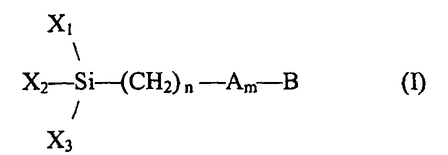 in which:
- n is comprised between 15 and 35,
- m is equal to 0 or to 1,
- X₁, X₂ and X₃, which can be identical or different among themselves, are chosen from among the group consisting of C₁ to C₆ saturated alkyl groups, straight-chain or branched, and hydrolysable groups, at least one of X₁, X₂ or X₃ representing a hydrolysable group,
- A represents the group -O-(CH₂-CH₂-O)ₖ-(CH₂)ᵢ- in which k is comprised between 0 and 100, and i represents an integer number greater than or equal to 0,
- in the case in which m = 0, then B represents an -OCOR, -OR or -COOR group or a halogen atom, R representing a hydrogen atom or C₁ to C₆ alkyl group, straight-chain or branched,
- in the case in which m = 1:
. if k = 0 and i = 0, then B represents an R₁ group,
. if k = 0 and i ≥ 1, then B represents an -OR₁, -OCOR₁, -NR₁R₂, -COOR₁, -CONR₁R₂ or -SR₁ group, or a halogen atom,
. if k ≥ 1 and i = 0, then B represents an R₁, -COR₁, -COOR₁, or -CONR₁R₂ group,
. if k ≥ 1 and i ≥ 1, then B represents an -OR₁, -OCOR₁, -NR₁R₂, -COOR₁, -CONR₁R₂ or -SR₁ group, or a halogen atom,
. R₁ and R₂, which can be identical or different, represent a hydrogen atom, an optionally substituted hydrocarbon chain, saturated or unsaturated and straight-chain or branched, comprising 1 to 24 carbon atoms, or an aromatic group.

2. Use according to Claim 1, **characterized in that** n is comprised between 20 and 25.

3. Use according to Claim 1 or Claim 2, **characterized in that** k is comprised between 0 and 5.

4. Use according to any of the foregoing Claims, **characterized in that** the said solid support is chosen from among the group consisting of glasses, ceramics, metals and metalloids.

5. Use according to any of the foregoing Claims, **characterized in that** the said hydrolysable group is chosen from among the group consisting of halogen atoms, the -N(CH₃)₂ group and the -OR' groups, R' being a saturated C₁ to C₆ alkyl group, straight-chain or branched.

6. Use according to any of the foregoing Claims, **characterized in that** X₁, X₂ and X₃ represent chlorine atoms, n is equal to 22, m is equal to 1, i is equal to 0, k is equal to 1 or 3 and B represents a -COCH₃ group.

7. Use according to any of the Claims 1 to 5, **characterized in that** X₁, X₂ and X₃ represent chlorine atoms, n is equal to 22, m is equal to I, i is equal to 1, k is equal to 2 and B represents a -COOCH₃ group.

8. Use according to any of the Claims 1 to 5, **characterized in that** X₁, X₂ and X₃ represent chlorine atoms, n is equal to 16, 22 or 27, m is equal to 0 and B represents an -OCOCH₃ group.

9. Use according to any of the Claims 1 to 5, **characterized in that** X₁, X₂ and X₃ represent chlorine atoms, n is equal to 21, m is equal to 0 and B represents a -COOCH₃ group.

10. Use according to any of the foregoing claims to prepare DNA chips.

11. Process to synthesise nucleic acids on a solid support, **characterized in that** the said support is modified by an organised, self-assembled mono-layer comprising at least one organo-silicon compound corresponding to Formula (I) as defined in any of the Claims 1 to 9, and **in that** it comprises the following stages:
a) preparation of a solid support modified by an organised, self-assembled mono-layer comprising at least one organo-silicon compound corresponding to Formula (I) as defined in any of the Claims 1 to 9, in which the said organo-silicon compounds have a protected hydroxyl or amine function at their end;
b) optionally, de-protection of the hydroxyl or amine function;
c) covalent coupling, in a localised manner, of a nucleotide onto the modified solid support obtained at stage a) or b);
d) repetition of stage c), at least once, with identical or different nucleotides.

12. Process according to Claim 11, **characterised in that** stage a) is performed by the following stages:
i) removal of contaminants from the solid support and hydration and/or hydroxylation of its surface,
j) introduction into a mixture of at least two solvents comprising at least one non-polar hydrocarbon solvent, under an inert atmosphere, of an organo-silicon compound of General Formula (I) as defined in any of the Claims 1 to 9, the said compound having a protected hydroxyl or amine function at one end,
k) silanisation of the support obtained at stage i) by immersion in the solution prepared in step j),
l) optionally, re-baking the silanised support obtained at stage k), carrying the self-assembled mono-layer, at a temperature comprised between 50 and 120°C, for a period of time of between 5 minutes and one night, and
m) rinsing the modified support obtained at stage k) or l) with a solvent.

13. Process according to Claim 11 or Claim 12, **characterized in that** a stage of grafting, at the end of the said organo-silicon compounds, of spacer arms carrying terminal hydroxyl or amine functions is performed after stage a) or b) and before stage c).

14. Process according to any of the Claims 11 to 13, **characterized in that** the nucleotides coupled in stage c) are protected, and **in that** stage d) is followed by a stage of de-protection of the nucleic acid that is synthesised.

15. Process to immobilise nucleic acids on a solid support, **characterized in that** the said support is modified by an organised, self-assembled mono-layer comprising at least one organo-silicon compound corresponding to Formula (I) as defined in any of the Claims 1 to 9, and **in that** it comprises the following stages:
a) preparation of a solid support modified by an organised, self-assembled mono-layer comprising at least one organo-silicon compound corresponding to Formula (I) as defined in any of the Claims 1 to 9, in which the said organo-silicon compounds have at their end a protected carboxylic acid or amine function;
b) optionally, de-protection of the carboxylic acid or amine function;
c) optionally, if the modified solid support carries terminal carboxylic acid functions, activation of these functions;
d) bringing the modified solid support obtained at stage a), b) or c) into contact with one or more locally applied solutions, in one or more polar solvents, of the nucleic acid(s) to be immobilised, the said nucleic acids having at one end a spacer arm functionalised either by an amine function if the modified solid support carries optionally protected terminal carboxylic acid functions, or by an activated carboxylic acid function if the modified solid support carries optionally protected terminal amine functions; and
e) washing the solid support onto which the said nucleic acids are immobilised.

16. Process according to Claim 15, **characterized in that** stage a) is performed by the following stages:
i) removal of contaminants from the solid support and hydration and/or hydroxylation of its surface,
j) introduction into a mixture of at least two solvents comprising at least one non-polar hydrocarbon solvent, under an inert atmosphere, of an organo-silicon compound of General Formula (I) as defined in any of the Claims 1 to 9, the said compound having a protected carboxylic acid or amine function at one end,
k) silanisation of the support obtained at stage i) by immersion in the solution prepared in step j),
l) optionally, re-baking the silanised support obtained at stage k), carrying the self-assembled mono-layer, at a temperature comprised between 50 and 120°C, for a period of time of between 5 minutes and one night, and
m) rinsing the modified support obtained at stage k) or l) with a solvent.

17. Process according to Claim 15 or Claim 16, **characterized in that** a stage of grafting, at the end of the said organo-silicon compounds, of spacer arms carrying terminal carboxylic acid or amine functions is performed after stage a) or b) and before stage c).

18. DNA chip, **characterized in that** it is obtained by the process according to any of the Claims 15 to 17, in which the said nucleic acids are DNA.
